(19) Europäisches Patentamt
European Patent Office
Office européen des brevets

(11) Numéro de publication: **0 360 685 B1**

(12) # FASCICULE DE BREVET EUROPEEN

(45) Date de publication de fascicule du brevet: **02.02.94**

(51) Int. Cl.5: **C07D 207/06**, C07D 211/14, C07D 211/22, C07D 207/08, A61K 31/40, A61K 31/44

(21) Numéro de dépôt: **89402561.8**

(22) Date de dépôt: **19.09.89**

Le dossier contient des informations techniques présentées postérieurement au dépôt de la demande et ne figurant pas dans le présent fascicule.

(54) **"[(diarylméthoxy) alcoyl] - 1 pyrrolidines et piperidines, procédés de préparation et médicaments les contenant".**

(30) Priorité: **23.09.88 FR 8812430**

(43) Date de publication de la demande:
**28.03.90 Bulletin 90/13**

(45) Mention de la délivrance du brevet:
**02.02.94 Bulletin 94/05**

(84) Etats contractants désignés:
**AT BE CH DE ES FR GB GR IT LI LU NL SE**

(56) Documents cités:

**CHEMICAL ABSTRACTS, vol. 101, 1984, Columbus, OH (US); E. LINDNER et al., p. 37, no. 65725p&NUM;**

(73) Titulaire: **LIPHA, LYONNAISE INDUSTRIELLE PHARMACEUTIOUE**
**34, rue Saint Romain**
**Boîte Postale 8481**
**F-69359 Lyon Cedex 08(FR)**

(72) Inventeur: **Ferrand, Gérard**
**62, rue des Aqueducs**
**F-69005 Lyon(FR)**
Inventeur: **Barbanton, Jacques**
**12, Domaine de la Côte**
**F-69530 Brignais(FR)**
Inventeur: **Depin, Jean-Claude**
**117, Cours Gambetta**
**F-69003 Lyon(FR)**
Inventeur: **Chavernac, Gilles**
**2 D, Chemin de la Bastero**
**F-60350 La Mulatière(FR)**

(74) Mandataire: **Moncheny, Michel et al**
**c/o Cabinet Lavoix**
**2 Place d'Estienne d'Orves**
**F-75441 Paris Cedex 09 (FR)**

EP 0 360 685 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne des nouvelles [(diarylméthoxy) alcoyl]-1 pyrrolidines et pipéridines répondant à la formule générale I.

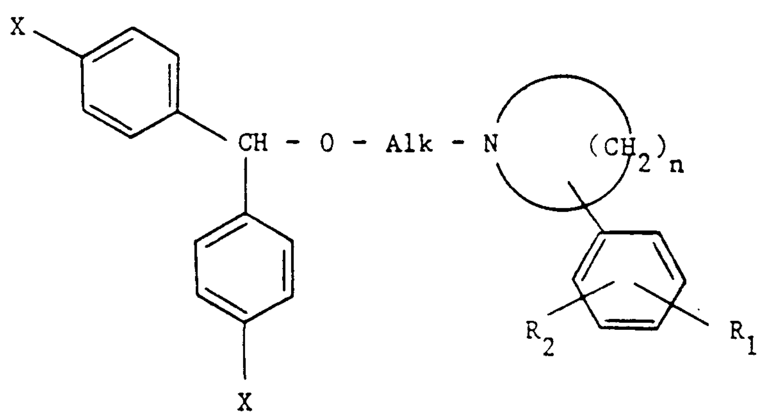

I

dans laquelle X est l'hydrogène ou un atome de fluor ; Alk est un groupe alcoyle linéaire ou ramifié contenant 2 ou 3 atomes de carbone ; $R_1$ et $R_2$ sont l'hydrogène ou un radical alcoxy linéaire ou ramifié comprenant de 1 à 4 atomes de carbone et pouvant occuper n'importe quelle position sur le noyau aromatique ; n peut avoir la valeur 4 ou 5.

Les composés de formule I pour lesquels Alk est un groupe alcoyle linéaire constituent une classe de composés particulièrement intéressante. Les sels pharmaceutiquement acceptables font partie intégrante de l'invention. Ce peuvent être des sels préparés soit à partir d'acides minéraux comme l'acide chlorhydrique, l'acide bromhydrique, l'acide sulfurique, l'acide phosphorique, soit à partir d'acides organiques comme l'acide tartrique, l'acide citrique, l'acide acétique, l'acide maléïque, l'acide fumarique, l'acide oxalique, l'acide méthanesulfonique.

Chemical Abstracts, vol. 101, 1984, résumé n°65725 p décrit le [[2-(diphénylméthoxy) éthyl-N-méthyl - amino] -2-méthyl éthyl] -3-méthoxy phényle et indique que celui-ci est un antagoniste des canaux calciques.

Les produits de l'invention peuvent se préparer selon la suite de réactions ci-dessous dans lesquelles X, $R_1$, $R_2$ et n ont les significations données précédemment.

Un bromodiphénylméthane de formule II est condensé avec un bromoalcanol de formule générale III. La réaction peut s'effectuer en présence d'un agent alcalin dans un solvant inerte ou bien en l'absence de solvant. De bons résultats ont été obtenus en opérant en présence d'un carbonate d'un métal alcalin comme le sodium ou le potassium à une température comprise entre 80 et 150 °C. Par condensation de l'halogénure obtenu de formule générale IV avec une arylpyrrolidine ou une arylpipéridine de formule générale V, on obtient les composés de l'invention. Cette dernière réaction s'effectue avantageusement en opérant dans un solvant inerte en présence d'une base. Les bases préférées sont les carbonates et les hydrures de métal alcalins tels que le sodium et le potassium. La température de la réaction peut être comprise entre la température ambiante et la température d'ébullition du solvant utilisé.

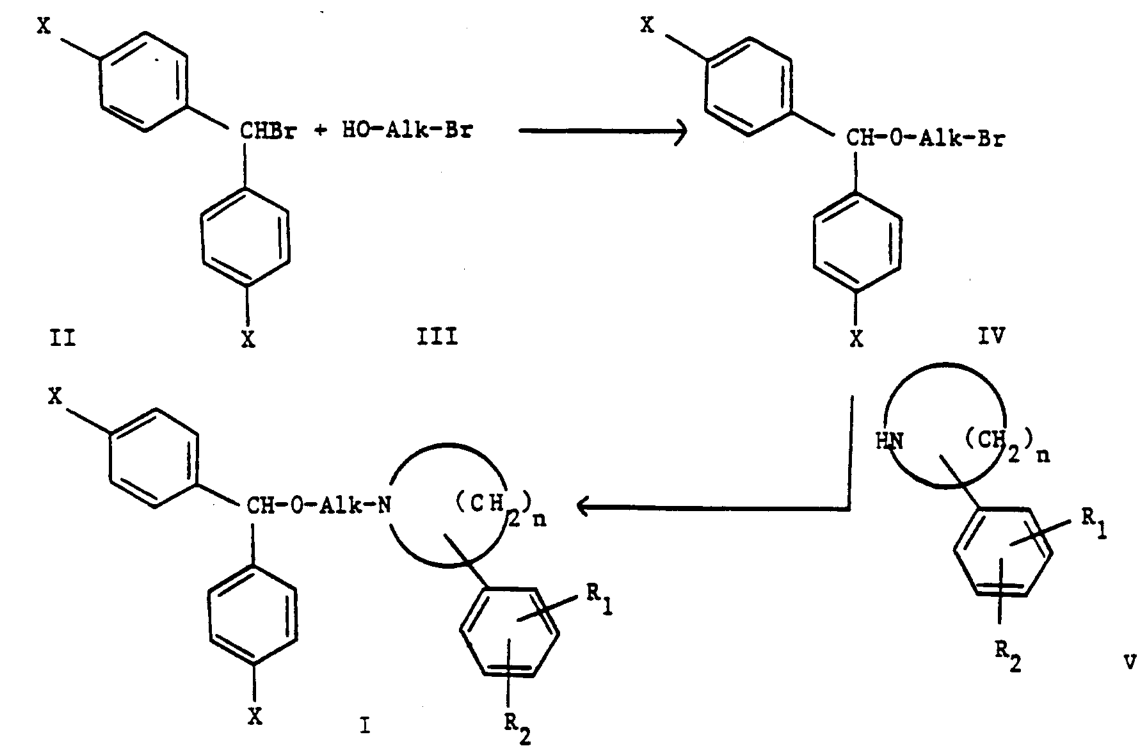

II III IV

I V

Les produits de l'invention pour lequels X est l'hydrogène et Alk est le radical éthylène $-CH_2-CH_2-$ peuvent également s'obtenir selon la séquence réactionnelle suivante dans laquelle $R_1$, $R_2$ et n ont les significations données précédemment :

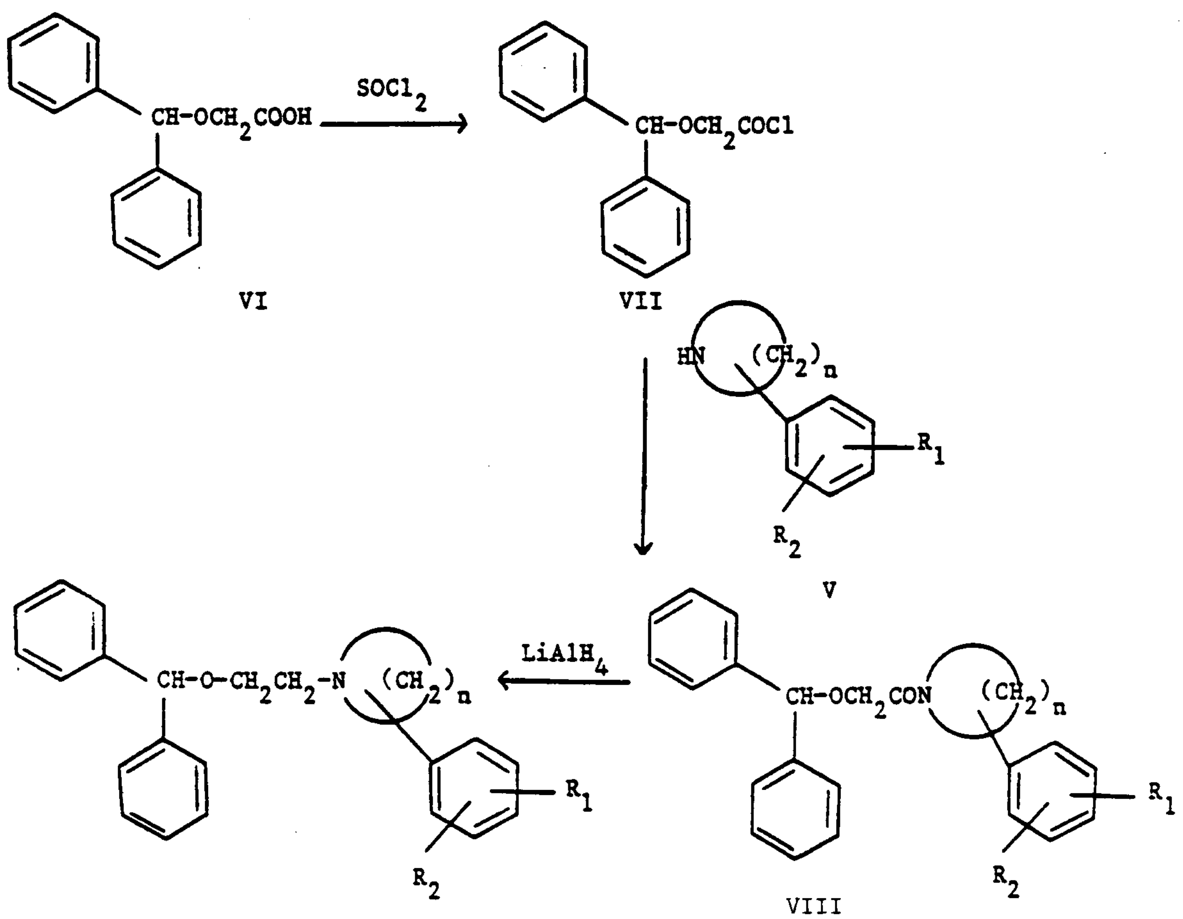


VI VII

V

VIII

L'acide diphénylméthoxyacétique VI traité par le chlorure de thionyle fournit le chlorure VII qui est condensé avec une arylpipéridine ou une arylpyrrolidine de formule générale V. Les aides VIII obtenus sont alors réduits par l'hydrure de lithium et d'aluminium. On opère en général avec un excès a d'agent réducteur, dans un solvant éthéré comme l'éther diéthylique ou le tétrahydrofuranne.

On a constaté que les composés représentés par la formule I possèdent de remarquables propriétés antagonistes du calcium qui les rendent utiles en médecine humaine, en particulier dans le traitement des maladies vasculaires périphériques et cérébrales, des désordres vestibulaires et dans la prophylaxie de la migraine.

L'effet inhibiteur calcique a été recherché in vitro par la méthode décrite par J.M. Van Nueten (Eur. J. Pharmacol., 1969, 6, 286) sur l'artère isolée dépolarisée de l'oreille de lapin.:
Un fragment de l'artère centrale de l'oreille est canulé et suspendu dans 100 ml d'une solution de Tyrode [NaCl : 136,90 mmoles/l, KCl : 2,68 mmoles/l. $CaCl_2$ : 3,60 mmoles/l, $MgCl_2$ : 1,04 mmole/l, $NaH_2PO_4$ : 0,42 mmole/l, glucose : 5,55 mmoles/l, $NaHCO_3$ : 11,90 mmoles/l]. Le fragment est oxygéné ($O_2$ : 95 %, $CO_2$ : 5 %) et perfusé par le tyrode à 37°C à débit constant à raison de 2 ml/minute. Les variations de pression sont enregistrées dans le circuit de perfusion par l'intermédiaire d'un capteur de pression et enregistrées en continu. Après une période d'équilibration, le tyrode est remplacé par une solution dépolarisante hyperpotassique [NaCl : 39,33 mmoles/l, KCl : 99,83 mmoles/l, $CaCl_2$ : 3,60 mmoles/l, $MgCl_2$ : 1,04 mmole/l, $NaH_2PO_4$ : 0,42 mmole/l, glucose : 5,55 mmoles/l, $NaHCO_3$ : 11,90 mmoles/l] entrainant par pénétration intracellulaire de calcium une vasoconstriction importante et durable antagonisée par un inhibiteur calcique.
Les produits de l'invention ont été testés à la dose unique de 10 µg introduite par injection dans le circuit de perfusion à raison de 0,1 ml pendant 10 secondes. Cette dose correspond à la dose abaissant durablement, d'environ 50%, la pression lors de l'injection de flunarizine prise comme étalon. L'effet des produits de l'invention sur la vasoconstriction indui-te par la solution hyperpotassique est suivi pendant plusieurs heures. L'intensité a été mesurée à l'acmé de leur action.

Chaque produit a été testé sur au moins trois fragments d'artère. Le tableau I regroupe les moyennes des diminutions de la pression de perfusion observées pour quelques produits de l'invention.

L'effet antagoniste du calcium des produits de l'invention est essentiellement intracellulaire. Ceci a été montré en étudiant leur action sur une protéine de transport du calcium,la calmoduline. Le principe de la méthode utilisée est l'activation qu'exerce la calmoduline sur l'activité enzymatique de base de la phosphodiéstérase cardiaque de type I (H.C. HO et Coll., Biochim. Biophys. Acta 1976, 429, 461). La mesure de l'activité enzymatique se fait selon la technique de W.J. THOMSON et H.M. APPLEMAN (Biochemistry, 1971, 10, 311). Dans ce test, les produits de l'invention manifestent une activité anti-calmoduline importante. Ainsi, le produit décrit dans l'exemple 10 possède une concentration inhibitrice 50 de 83 µM alors que dans les mêmes conditions l'inhibition obtenue avec la flunarizine n'est que de 6 % à 100 µM.

TABLEAU I

| PRODUITS | CHUTE % DE LA PRESSION DE PERFUSION ± ERREUR STANDARD |
|---|---|
| EXEMPLE 1 | 56 ± 6 |
| EXEMPLE 4 | 54 ± 5 |
| EXEMPLE 10 | 59 ± 4 |
| EXEMPLE 11 | 56 ± 5 |
| EXEMPLE 13 | 58 ± 5 |
| EXEMPLE 14 | 54 ± 5 |
| EXEMPLE 15 | 52 ± 5 |
| FLUNARIZINE | 41 ± 4 |

Les composés de la présente invention sont peu toxiques. Ainsi, le composé de l'exemple 10 a une dose létale 50 chez le rat supérieure à 1 g/kg quand on l'administre par voie orale.

La présente demande a également pour objet l'application des composés I à titre de médicaments et notamment de médicaments cardiovasculaires. Ces médicaments peuvent être administrés par voie orale

sous forme de comprimés,comprimés dragéïfiés ou de gélules ou par voie intraveineuse sous forme de soluté injectable. Le principe actif est associé à divers excipients pharmaceutiquement compatibles. Les posologies journalières peuvent varier de 1mg à 100 mg de principe actif selon l'âge du patient et la gravité de l'affection traitée.

On donne ci-dessous, à titre d'exemple non limitatif, quelques formulations pharmaceutiques :

| - Composition d'un comprimé de 100mg éventuellement enrobé : | |
|---|---|
| • principe actif | 10 mg, |
| • lactose | 40 mg, |
| • amidon de blé | 37 mg, |
| • gélatine | 2 mg, |
| • acide alginique | 5 mg, |
| • talc | 5 mg, |
| • stéarate de magnésium | 1 mg. |

| - Composition d'une gélule : | |
|---|---|
| • principe actif | 10 mg, |
| • lactose | 32 mg, |
| • amidon de blé | 25 mg, |
| • talc | 2,5 mg, |
| • stéarate de magnésium | 0,5 mg. |

Les exemples suivants illustrent l'invention à titre non limitatif.

EXEMPLE 1

[(Diphénylméthoxy)-2 éthyl]-1 (méthoxy-3 phényl)-3 pyrrolidine, oxalate

a) (Diphénylméthoxy)-1 bromo-2 éthane

On ajoute 37,5g (0,3 mole) de bromo-2 éthanol à un mélange de 61,8g (0,25 mole) de bromodiphénylméthane et de 31,8 g (0,3 mole) de carbonate de sodium. Le milieu réactionnel est porté lentement à 130 °C, maintenu 5h à cette température puis abandonné une nuit à température ambiante. Le mélange est repris à l'éther et lavé à l'eau. L'extrait éthéré est séché sur sulfate de sodium. L'évaporation de l'éther fournit une huile qui est purifiée par distillation. Rdt : 55 g (75 %). $Eb_{0,25-0,3}$ = 128 - 135 °C.

b) [(Diphénylméthoxy)-2 éthyl]-1(méthoxy-3 phényl)-3 pyrrolidine,oxalate

On ajoute une solution de 17,5 g (60 mmoles) de (diphénylméthoxy)-1 bromo-2 éthane dans 50 $cm^3$ de diméthylformamide à un mélange de 10,6g (60 mmoles) de (méthoxy-3 phényl)-3 pyrrolidine [préparée selon A. Ebnöther et K. Hasspacher, brevet suisse 526 536], de 16,6 g (120 mmoles) de carbonate de potassium, de 300 $cm^3$ de diméthylformamide et de quelques cristaux d'iodure de potassium. Le mélange est porté 13h à 80 °C puis après retour à température ambiante, filtré et concentré à sec sous pression réduite. Le résidu est repris par 500$cm^3$ d'acide chlorhydrique 1N et extrait à l'éther. La phase aqueuse acide est séparée, basifiée par de la soude 10 N et extraite au chlorure de méthylène. La solution organique est séchée sur sulfate de sodium. L'évaporation du solvant fournit un résidu huileux qui est purifié par filtration sur silice (éluant hexane-acétate d'éthyle 1/1). On obtient 10 g de [(diphénylméthoxy)-2 éthyl]-1 (méthoxy-3 phényl)-3 pyrrolidine que l'on dissout dans 100 $cm^3$ d'éther. A cette solution on ajoute une solution de 2,55 g (28,3 mmoles) d'acide oxalique dans 30 $cm^3$ d'acétone. Le précipité d'oxalate est filtré et recristallisé dans l'acétone. Rdt : 9,0 g (31 %). F = 133 - 134 °C.

| Analyse centésimale : $C_{28}H_{31}NO_6$ (M = 477,56) | | | |
|---|---|---|---|
| | C% | H % | N % |
| Calculé<br>Trouvé | 70,42<br>70,53 | 6,54<br>6,71 | 2,93<br>2,87 |

R.M.N. (DMSO $d_6$)
δ = 3,0-3,9 [avec pic à 3,7] (massif complexe, 14H); 5,5(singulet,1H); 6,6 (pic élargi, 2H, échangeables par CF3COOD) ; 6,7-7,6 (multiplet 14 H).

EXEMPLE 2

[(Diphénylméthoxy)-2 éthyl]-1 (méthoxy-3 phényl)-3 pyrrolidine, chlorhydrate

On ajoute 1,35 $cm^3$ d'une solution 7,4 M de gaz chlorhydrique dans l'éthanol à une solution de 3,9g de [(diphénylméthoxy)-2 éthyl]-1 (méthoxy-3 phényl)-3 pyrrolidine [préparée selon l'exemple 1 ci-dessus] dans 100$cm^3$ d'éther. Le précipité est filtré et recristallisé dans l'acétone. Rdt : 1,8g (42%). F = 118 - 120 °C.

| Analyse centésimale : $C_{26}H_{30}ClNO_2$ (M = 423,98) | | | | |
|---|---|---|---|---|
| | C % | H % | Cl % | N % |
| Calculé<br>Trouvé | 73,66<br>73,36 | 7,13<br>7,10 | 8,36<br>8,44 | 3,30<br>3,32 |

EXEMPLE 3

[(Diphénylméthoxy)-2 éthyl]-1 (méthoxy-3 phényl)-4 pipéridine, oxalate

En opérant comme dans l'exemple 1 à partir de 5,8g (20 mmoles) de (diphénylméthoxy)-1 bromo-2 éthane, de 3,8g (20 mmoles) de (méthoxy-3 phényl)-4 pipéridine [préparée selon la méthode de B.M. Iselin et K. Hoffmann, Helv. Chim. Acta 1954, 37, 178] et de 5,5g (40 mmoles) de carbonate de potassium, on obtient après filtration sur colonne de silice et passage à l'oxalate 4,9 g (Rdt 50 %) du produit cherché.
F = 145 - 146 °C (acétone-éthanol)

| Analyse centésimale : $C_{29}H_{33}NO_6$ (M = 491,58) | | | |
|---|---|---|---|
| | C % | H % | N % |
| Calculé<br>Trouvé | 70,86<br>70,66 | 6,77<br>6,76 | 2,85<br>2,73 |

EXEMPLE 4

(Diméthoxy-3,4 phényl)-3 [(diphénylméthoxy)-2 éthyl]-1 pyrrolidine, oxalate

En opérant comme dans l'exemple 1 à partir de 14,5 g (49,7 mmoles) de (diphénylméthoxy)-1 bromo-2 éthane, de 10,3 g (49,7 mmoles) de (diméthoxy-3,4-phényl)-3 pyrrolidine [préparée selon A. Ebnöther et K. Hasspacher, brevet suisse 526 536] et de 13,8g (100 mmoles) de carbonate de potassium, on obtient après filtration sur colonne de silice (éluant hexane-acétate d'éthyle 1/1) 10,1g (rdt 49 %) de (diméthoxy-3,4 phényl)-3 [(diphénylméthoxy)-2 éthyl]-1 pyrrolidine sous forme d'une huile épaisse.

A une solution de 8,5g (20,3 mmoles) de base dans 40 $cm^3$ d'acétone, on ajoute une solution de 1,8g d'acide oxalique dans 10 $cm^3$ d'acétone. Le précipité d'oxalate est filtré et recristallisé dans l'éthanol. On obtient 7,8 g (rdt global 37 %) d'oxalate de (diméthoxy-3,4 phényl)-3 [(diphénylméthoxy)-2 éthyl]-1 pyrrolidine. F = 165 - 167 °C.

| Analyse centésimale : $C_{29}H_{33}NO_7$ (M = 507,58) | | | |
|---|---|---|---|
| | C % | H % | N % |
| Calculé<br>Trouvé | 68,62<br>68,36 | 6,55<br>6,38 | 2,76<br>2,79 |

EXEMPLE 5

[(Diphénylméthoxy)-2 éthyl]-1 phényl-3 pyrrolidine, chlorhydrate

En opérant comme dans l'exemple 1, à partir de 14,6g (50 mmoles) de (diphénylméthoxy)-1 bromo-2 éthane, de 7,3 g (50 mmoles) de phényl-3 pyrrolidine et de 13,8g (100 mmoles) de carbonate de potassium, on obtient, après filtration sur colonne de silice (éluant hexane-acétate d'éthyle 1/1) 11 g (Rdt 61,5 %) de [(diphénylméthoxy)-2 éthyl]-1 phényl-3 pyrrolidine, sous forme d'une huile épaisse.

A une solution de 9g (25,2 mmoles) de base dans 150 $cm^3$ d'éther, on ajoute 3,7 $cm^3$ d'une solution 7,4 M de gaz chlorhydrique dans l'éthanol. Le précipité de chlorhydrate est filtré et recristallisé dans l'acétone. On obtient 5,7g (rendement global 35 %) de chlorhydrate de [(diphénylméthoxy)-2 éthyl]-1 phényl-3 pyrrolidine. F = 131 - 132 °C.

| Analyse centésimale : $C_{25}H_{28}ClNO$ (M = 393,96) | | | | |
|---|---|---|---|---|
| | C % | H % | Cl % | N % |
| Calculé<br>Trouvé | 76,22<br>76,03 | 7,16<br>7,19 | 9,00<br>9,05 | 3,56<br>3,62 |

EXEMPLE 6

[(Diphénylméthoxy)-3 propyl]-1 (méthoxy-3 phényl)-3 pyrrolidine, chlorhydrate

a) (Diphénylméthoxy)-1 bromo-3-propane

Un mélange de 12,3 g (50 mmoles) de bromodiphénylméthane, de 8,3 g (60 mmoles) de bromo-3 propanol et de 6,3g (60 mmoles) de carbonate de sodium est porté à 120-130 °C pendant 10h puis abandonné une nuit à température ambiante. Le mélange est repris à l'éther et lavé à l'eau. L'extrait éthéré est séché sur sulfate de sodium. L'avaporation de l'éther fournit une huile qui est purifiée par distillation. Rdt : 9,1g (60 %). $Eb_{0,25}$ = 130 - 140 °C.

b) [(Diphénylméthoxy)-3 propyl]-1 (méthoxy-3 phényl)-3 pyrrolidine, chlorhydrate.

Une solution de 15,3g (50 mmoles) de (diphénylméthoxy)-1 bromo-3 propane dans 50$cm^3$ de diméthylformamide est ajoutée à un mélange de 8,8g (50 mmoles) de (méthoxy-3 phényl)-3 pyrrolidine, de 13,8g (100 mmoles) de carbonate de potassium, de quelques cristaux d'iodure de potassium et de 250$cm^3$ de diméthylformamide. Le milieu réactionnel est porté 12h à 80 °C puis filtré après retour à température ambiante. Le filtrat est concentré à sec sous pression réduite. Le résidu est repris à l'éther et extrait avec 500$cm^3$ d'acide chlorhydrique N. La phase aqueuse est séparée, basifiée par une solution de soude 10 N et extraite au chlorure de méthylène. La solution organique est séchée sur sulfate de sodium. L'évaporation du solvant fournit un résidu huileux qui est purifié par filtration sur colonne de silice (éluant hexane-acétate d'éthyle 1/1). On obtient 10,3 g (rdt 51,3 %) de [(diphénylméthoxy)-3 propyl]-1 (méthoxy-3 phényl)-3 pyrrolidine.

A une solution de 9g de base dans 150$cm^3$ d'ether, on ajoute une solution 7,4 M de gaz chlorhydrique dans l'éthanol. Le précipité est filtré et recristallisé dans l'acétate d'éthyle. On obtient 7,4 g (rendement global 39 %) de chlorhydrate de [(diphénylméthoxy)-3 propyl]-1 (méthoxy-3 phényl)-3 pyrrolidine. F = 109 - 110 °C.

| Analyse centésimale : $C_{27}H_{32}ClNO_2$ (M = 438,01) | | | | |
|---|---|---|---|---|
| | C % | H % | Cl % | N % |
| Calculé<br>Trouvé | 74,04<br>73,85 | 7,36<br>7,43 | 8,09<br>8,14 | 3,20<br>2,96 |

R.M.N. ($CDCl_3$)
δ = 2,0-2,7 (massif complexe, 4H) ; 2,9-4,2 (multiplet, 12H); 5,3 (singulet, 1H) ; 6,6-7,5 (multiplet, 14H).

EXEMPLE 7

[(Diphénylméthoxy)-3 propyl-2]-1 (méthoxy-3 phényl)-3 pyrrolidine, oxalate

a) (Diphénylméthoxy)-1 bromo-2 propane

Un mélange de 12,4 g (50 mmoles) de bromodiphénylméthane, de 6,4g (60 mmoles) de carbonate de sodium et de 8,3g (60 mmoles) de bromo-2 propanol [préparé selon R.F. Nystrom, J. Am. Chem. Soc. 1959, 81, 610] est porté en 2h à 130 °C et maintenu 8h à cette température. Après retour à température ambiante, le milieu réactionnel est repris à l'éther et lavé à l'eau. La phase éthérée est séparée puis le solvant est évaporé. La distillation du résidu fournit le (diphénylméthoxy)-1 bromo-2 propane utilisé dans la suite sans autre purification. Rdt : 10,5g (69 %). $Eb_{0,4}$ = 120 - 130 °C.

b) [(Diphénylméthoxy)-3 propyl-2]-1 (méthoxy-3 phényl)-3 pyrrolidine, oxalate

Une solution de 10,5g (34,4 mmoles) de (diphénylméthoxy)-1 bromo-2 propane dans 50 $cm^3$ de diméthylformamide est ajoutée rapidement à un mélange de 6,1g (34,4 mmoles) de (méthoxy-3 phényl)-3 pyrrolidine, de 9,5g (68,8mmoles) de carbonate de potassium, de 150$cm^3$ de diméthylformamide et de quelques cristaux d'iode. Le milieu réactionnel est agité 14h à 60 °C. Les insolubles minéraux sont filtrés puis la solution est concentrée à sec sous pression réduite. Le résidu est repris par de l'acide chlorhydrique 5N et lavé à l'éther. La phase aqueuse est séparée puis basifiée par de la soude 5N. Le mélange est extrait au chlorure de méthylène. La solution organique est lavée à l'eau, séchée sur sulfate de sodium et concentrée à sec. Le résidu huileux est filtré sur colonne de silice (éluant hexane-acétate d'éthyle 1/1). On obtient 3,9 g d'une huile que l'on dissout dans 100 $cm^3$ d'éther. On ajoute à cette solution une solution de 0,96 g (10,6 mmoles) d'acide oxalique dans 20 $cm^3$ d'acétone. L'oxalate de [(diphénylméthoxy)-3 propyl-2]-1 (méthoxy-3 phényl)-3 pyrrolidine est filtré et recristallisé dans l'acétone. Rdt : 2,2 g (11%). F = 119 - 121 °C.

| Analyse centésimale : $C_{29}H_{33}NO_6$ (M = 491,58) | | | |
|---|---|---|---|
| | C % | H % | N % |
| calculé<br>Trouvé | 70,86<br>70,80 | 6,77<br>6,57 | 2,85<br>3,00 |

R.M.N. ($CDCl_3$)
δ = 1,4 (doublet, 2H) ; 2,0-2,7 (massif complexe, 2H) ; 2,9-4,2 (massif complexe, 10H dont 2 échangeables par $CF_3COOD$) ; 5,3 (singulet, 1H) ; 6,6-7,7 (multiplet, 14H).

EXEMPLE 8

[(Diphénylméthoxy)-2 éthyl]-1 phényl-2 pyrrolidine, oxalate

En opérant comme dans l'exemple 1 à partir de 14,6 g (50 mmoles) de (diphénylméthoxy)-1 bromo-2 éthane, de 7,3 g (50 mmoles) de phényl-2 pyrrolidine et de 13,8 g (100 mmoles) de carbonate de potassium on obtient, après filtration sur colonne de silice, 9 g (Rdt 50 %) de [(diphénylméthoxy)-2éthyl]-1 phényl-2 pyrrolidine.

L'oxalate est préparé de manière habituelle. F = 90 - 94 °C (acétone).

| Analyse centésimale $C_{27}H_{29}NO_5$ (M = 447,53) | | | |
|---|---|---|---|
| | C % | H % | N % |
| Calculé | 72,46 | 6,53 | 3,13 |
| Trouvé | 72,73 | 6,30 | 2,95 |

EXEMPLE 9

[[Bis (fluoro-4 phényl) méthoxy]-2 éthyl]-1 (méthoxy-3 phényl)-3 pyrrolidine, oxalate

a) [Bis (fluoro-4 phényl) méthoxy]-1 bromo-2 éthane

Un mélange de 49,5 g (0,175 mole) de bromo bis (fluoro-4 phényl) méthane, de 22,4g (0,21 mole) de carbonate de sodium et de 26,9 g (0,21 mole) de bromo-2 éthanol est agité à 130 °C pendant 15 heures. Après refroidissement, le milieu réactionnel est repris à l'eau et extrait à l'éther. La phase éthérée est lavée, séchée sur sulfate de sodium puis concentrée à sec. La distillation du résidu fourni 41,9 g (rdt : 69,4 %) de [bis(fluoro-4 phényl) méthoxy]-1 bromo-2 éthane. $Eb_{0,2}$ = 119 - 123 °C.

b) [[Bis (fluoro-4 phényl) méthoxy]-2 éthyl]-1 (methoxy-3 phényl)-3 pyrrolidine, oxalate

Une solution de 7,2 g (0,022 mole) de [bis (fluoro-4 phényl) méthoxy]-1 bromo-2 éthane dans 20 $cm^3$ de diméthylformamide est ajoutée rapidement à un mélange de 3,9 g (0,022 mole) de (méthoxy-3 phényl)-3 pyrrolidine, de 6,1 g (0,044 mole) de carbonate de potassium et de 110 $cm^3$ de diméthylformamide. Le mélange est agité 16h à 80 °C, filtré puis concentré sous pression réduite. Le résidu est repris à l'éther et extrait par une solution d'acide chlorhydrique 2N. La phase aqueuse acide est lavée à l'éther, alcalinisée par une solution de soude 10N et extraite au chlorure de méthylène. La solution organique est lavée à l'eau, séchée sur sulfate de sodium et concentrée à sec. On obtient 9,2 g d'un résidu huileux qui est purifié par flash-chromatographie sur silice (éluant hexane-acétate d'éthyle 1/1). On obtient 4,5 g (Rdt = 48 %) de [-[bis (fluoro-4 phényl) méthoxy]-2 éthyl]-1 (méthoxy-3 phényl)-3 pyrrolidine.

Le passage à l'oxalate se fait en traitant une solution de 4,3 g de la base obtenue ci-dessus dans 30 $cm^3$ d'acétone par une solution de 0,9 g d'acide oxalique dans 10 $cm^3$ d'acétone. Le précipité d'oxalate est filtré et recristallisé dans l'acétone. Rdt : 3,5 g. F = 133 - 135 °C.

| Analyse centésimale : $C_{28}H_{29}F_2NO_6$ (M = 513,54) | | | | |
|---|---|---|---|---|
| | C % | H % | F % | N % |
| Calculé | 65,49 | 5,69 | 7,40 | 2,73 |
| Trouvé | 65,37 | 5,73 | 7,30 | 3,01 |

R.M.N. (DMSO $d_6$) :
$\delta$ = 1,7 - 2,6 ppm (massif complexe, 2H) : 3,0 - 4,0 ppm [avec pic à 3,7 ppm] (massif complexe, 12H) ; 5,6 ppm (singulet, 1H) ; 6,7 - 7,6 ppm (multiplet, 12H) ; 11,1 ppm (pic élargi, 2H, échangeable à l'acide trifluoro-acétique).

EXEMPLE 10 :

[[Bis (fluoro-4 phényl) méthoxy]-2 éthyl]-1 méthoxy-3-phényl)-3 pyrrolidine, fumarate

A une solution de 120,2 g (0,678 mole) de (méthoxy-3 phényl)-3 pyrrolidine dans 2 l de diméthylformamide, on ajoute par portions 29,8 g (0,746 mole) d'une suspension à 60 % d'hydrure de sodium dans l'huile. Le mélange est porté à 45 °C pendant 1 heure. On ajoute une solution de 244,1 g (0,746 mole) de [bis (fluoro-4 phényl) méthoxy]-1 bromo-2 éthane (préparé selon l'exemple 9) dans 400 $cm^3$ de diméthylformamide et porte le mélange à 80 °C pendant 9 heures. On rajoute 24,4 g de [bis (fluoro-4 phényl) méthoxy]-1 bromo-2 éthane et poursuit le chauffage à 80 °C pendant 4 heures. Le milieu réactionnel est concentré sous pression réduite, repris à l'eau et extrait au chlorure de méthylène. La phase organique est

séparée, lavée à l'eau et séchée sur sulfate de sodium. L'évaporation du solvant fournit une huile qui est purifiée par filtration sur colonne de silice (éluant acétate d'éthyle). La [[bis (fluoro-4 phényl) méthoxy]-2 éthyl]-1 (méthoxy-3 phényl)-3 pyrrolidine obtenue est mise en solution dans 500 $cm^3$ d'éthanol.

On ajoute une solution de 69,8 g (0,602 mole) d'acide fumarique dans 150$cm^3$ d'éthanol et concentre à sec. Le résidu est trituré dans l'éther, filtré et recristallisé dans un mélange éthanol-eau. On obtient 216,2 g (rdt : 59 %) de fumarate. F = 142 - 144 °C.

| Analyse centésimale : $C_{30}H_{31}F_2NO_6$ | | | | |
|---|---|---|---|---|
| | C % | H % | F % | N % |
| Calculé | 66,78 | 5,79 | 7,04 | 2,60 |
| Trouvé | 67,15 | 5,77 | 7,00 | 2,65 |

EXEMPLE 11

(Diphénylméthoxy-2 éthyl)-1 (méthoxy-3 phényl)-2 pyrrolidine, fumarate

En opérant comme dans l'exemple 1 à partir de 8,7 g (30 mmoles) de (diphénylméthoxy)-1 bromo-2 éthane, de 5,3 g (30 mmoles) de (méthoxy-3 phényl)-2 pyrrolidine [préparée selon J.H. Burckhalter et J.E. Short, J. Org. Chem. 1958, 23, 1281] et de 8,3 g (60 mmoles) de carbonate de potassium, on obtient après filtration sur colonne de silice 6,2 g (rdt : 53 %) de (diphénylméthoxy-2 éthyl)-1 (méthoxy-3 phényl)-2 pyrrolidine. Le fumarate est préparé de manière habituelle. F = 134 - 135 °C (acétone-éthanol).

| Analyse centésimale : $C_{30}H_{33}NO_6$ (M = 503,60) | | | |
|---|---|---|---|
| | C % | H % | N % |
| Calculé | 71,55 | 6,61 | 2,78 |
| Trouvé | 71,80 | 6,58 | 2,85 |

EXEMPLE 12

[[Bis (fluoro-4 phényl) méthoxy]-3 propyl]-1 (méthoxy-3 phényl)-3 pyrrolidine, fumarate

a) [Bis (fluoro-4 phényl) méthoxy]-1 bromo-3 propane

Un mélange de 36,9 g (0,130 mole) de bromo bis (fluoro-4 phényl) méthane, de 21,7 g (0,156 mole) de bromo-3 propanol, de 16,6 g (0,156 mole) de carbonate de sodium et de quelques cristaux d'iodure de potassium est porté à 130 °C pendant 16 heures. Après refroidissement, le milieu réactionnel est repris à l'eau et extrait à l'éther. La phase éthérée est lavée à l'eau, séchée sur sulfate de sodium puis concentrée à sec. La distillation du résidu fournit 30,5 g (rdt : 68 %) de [bis (fluoro-4 phényl) méthoxy]-1 bromo-3 propane. $Eb_{0,15}$ = 134 - 140 °C.

b) [[Bis (fluoro-4 phényl méthoxy]-3 propyl]-1 (méthoxy-3 phényl)-3 pyrrolidine, fumarate

Une solution de 17 g (50 mmoles) de [bis (fluoro-4 phényl) méthoxy]-1 bromo-3 propane dans 50 $cm^3$ de DMF est ajoutée rapidement à un mélange de 8,8 g (50 mmoles) de (méthoxy-3 phényl)-3 pyrrolidine, de 13,8 g (100 mmoles) de carbonate de potassium et de 250 $cm^3$ de DMF. Le mélange est agité 10 heures à 80 °C, filtré puis concentré sous pression réduite. Le résidu est repris à l'éther puis extrait par une solution d'acide chlorhydrique N. La phase aqueuse acide est lavée à l'éther, alcalinisée par une solution de soude 10N et extraite au chlorure de méthylène. La solution organique est lavée à l'eau, séchée sur sulfate de sodium et concentrée à sec pour donner 17,4 g d'une huile qui est purifiée par flash chromatographie sur silice (éluant hexane-acétate d'éthyle 1/1). On obtient 12,3 g (rdt : 56 %) de [[bis (fluoro-4 phényl) méthoxy]-3 propyl]-1 (méthoxy-3 phényl )-3 pyrrolidine.

Le fumarate est préparé de manière habituelle. F = 103 - 104 °C (isopropanol)

| Analyse centésimale : $C_{31}H_{33}F_2NO_6$ (M = 553,60) | | | | |
|---|---|---|---|---|
| | C % | H % | F % | N % |
| Calculé | 67,26 | 6,01 | 6,86 | 2,53 |
| Trouvé | 67,55 | 6,12 | 6,82 | 2,82 |

EXEMPLE 13

[(Diphénylméthoxy)-2 éthyl]-1 (méthoxy-2 phényl)-3 pyrrolidine, maléate

En opérant comme dans l'exemple 1 à partir de 17,5 g (60 mmoles) de (diphénylméthoxy)-1 bromo-2 éthane, de 10,6 g (60 mmoles) de (méthoxy-2 phényl)-3 pyrrolidine [J.R. Dice et D.V. Lopiekes brevet US 2.975.193] et de 16,6 g (120 mmoles) de carbonate de potassium on obtient après filtration sur colonne de silice et passage au maléate 7,1 g (rdt : 24 %) du produit cherché. F = 97 - 99 ° C (acétone - éther).

| Analyse centésimale : $C_{30}H_{33}NO_6$ (M = 503,60) | | | |
|---|---|---|---|
| | C % | H % | N % |
| Calculé | 71,55 | 6,61 | 2,78 |
| Trouvé | 71,53 | 6,53 | 2,71 |

EXEMPLE 14

[[Bis (fluoro-4 phényl) méthoxy]-2 éthyl]-1 (isopropoxy-3 phényl)-3 pyrrolidine, fumarate

En opérant comme dans l'exemple 9, à partir de 16,4 g (50 mmoles) de [bis (fluoro-4 phényl) méthoxy]-1 bromo-2 éthane, de 10,3 g (50 mmoles) d'(isopropoxy-3 phényl)-3 pyrrolidine et de 13,8 g (100 mmoles) de carbonate de potassium, on obtient après filtration sur colonne de silice 16,4 g (rdt : 73 %) de [[bis (fluoro-4 phényl) méthoxy]-2 éthyl]-1 (isopropoxy-3 phényl)-3 pyrrolidine.

Le fumarate est préparé de manière habituelle. F = 121 - 124 ° C (éthanol-eau).

| Analyse centésimale : $C_{32}H_{35}F_2NO_6$ (M = 567,63) | | | | |
|---|---|---|---|---|
| | C % | H % | F % | N % |
| Calculé | 67,71 | 6,22 | 6,69 | 2,47 |
| Trouvé | 67,65 | 6,20 | 6,66 | 2,40 |

L'(isopropoxy-3 phényl)-3 pyrrolidine est préparée selon la méthode décrite par A. Ebnöther et K. Hasspacher(brevet suisse 526536) en partant d'isopropoxy-3 benzaldéhyde. $Eb_{0,5}$ = 110 - 114 ° C.

EXEMPLE 15

[[Bis (fluoro-4 phényl) méthoxy]-2 éthyl]-1 (diméthoxy-3,5 phényl)-3 pyrrolidine, fumarate

En opérant comme dans l'exemple 9 à partir de 14,8 g (45 mmoles) de [bis (fluoro-4 phényl) méthoxy]-1 bromo-2 éthane, de 9,4 g (45 mmoles) de(diméthoxy-3,5 phényl)-3 pyrrolidine et de 12 g (86 mmoles) de carbonate de potassium, on obtient après chromatographie sur colonne de silice (éluant chloroforme-méthanol 9/1) 9,5 g (rdt : 46 %) de [[Bis (fluoro-4 phényl) méthoxy]-2 éthyl]-1 (diméthoxy-3,5 phényl)-3 pyrrolidine.

Le fumarate est préparé de manière habituelle. F = 106 - 108 ° C (acétone - éther).

| Analyse centésimale : $C_{31}H_{33}F_2NO_7$ (M = 569,60) | | | | |
|---|---|---|---|---|
| | C % | H % | F % | N % |
| Calculé | 65,37 | 5,84 | 6,67 | 2,46 |
| Trouvé | 65,35 | 5,82 | 6,57 | 2,40 |

La (diméthoxy-3,5 phényl)-3 pyrrolidine est préparée selon la méthode de A. Ebnöther et K. Hasspacher (brevet suisse 526536) en partant de diméthoxy-3,5 benzaldéhyde. $Eb_{0,4}$ = 132 - 138 ° C.

EXEMPLE 16

[[Bis (fluoro-4 phényl)méthoxy]-2 éthyl]-1 (méthoxy-3 phényl)-2 pyrrolidine, fumarate

En opérant comme dans l'exemple 9 à partir de 9,8 g (30 mmoles) de [bis (fluoro-4 phényl)méthoxy]-1 bromo-2 éthane, de 5,3 g (30 mmoles) de (méthoxy-3 phényl)-2 pyrrolidine et de 8,3 g (60 mmoles) de carbonate de potassium, on obtient après filtration sur colonne de silice 6,7 g (rdt : 52 %) de [[bis (fluoro-4 phényl)méthoxy]-2 éthyl]-1 (méthoxy-3 phényl)-2 pyrrolidine.

Le fumarate est préparé de manière habituelle. F = 134 - 136 ° C (acétone).

| Analyse centésimale : $C_{30}H_{31}F_2NO_6$ (M = 539,58) | | | | |
|---|---|---|---|---|
| | C % | H % | F% | N % |
| Calculé | 66,78 | 5,79 | 7,04 | 2,60 |
| Trouvé | 66,49 | 5,88 | 7,03 | 2,45 |

EXEMPLE 17

[(Diphénylméthoxy)-2 éthyl]-1 phényl-3 pipéridine, oxalate

a) Chlorure de (diphénylméthoxy) acétyle

On ajoute 8,1 g (68,1 mmoles) de chlorure de thionyle à une solution de 5,5 g (22,7 mmoles) d'acide (diphénylméthoxy) acétique dans 150 $cm^3$ de benzène. La solution est portée 5 heures à reflux puis évaporée à sec sous pression réduite. La distillation du résidu fournit 4,3 g (rdt : 73%) de chlorure de (diphénylméthoxy) acétyle. $Eb_{0,25}$ = 115 - 125 ° C.

b) [(Diphénylméthoxy) acétyl]-1 phényl-3 pipéridine

Une solution de 4,5 g (17,2 mmoles) de chlorure de (diphénylméthoxy) acétyle dans 50 $cm^3$ de chlorure de méthylène est ajoutée goutte à goutte, à température ambiante, à une solution de 2,8 g (17,2 mmoles) de phényl-3 pipéridine [préparée selon M. Julia et coll., Bull. Soc. Chim. Fr. 1968, 987] et de 3,5 g (34,4 mmoles) de triéthylamine dans 100 $cm^3$ de chlorure de méthylène. Le milieu réactionnel est porté 4 heures à reflux, laissé au repos une nuit puis versé dans une solution de 10 $cm^3$ d'acide chlorhydrique concentré dans 300 $cm^3$ d'eau. Le mélange est extrait au chlorure de méthylène, lavé à l'eau et séché sur sulfate de sodium. Après évaporation du solvant sous pression réduite, le résidu est purifié par filtration sur silice (éluant hexane-acétate d'éthyle1/1) puis par recristallisation dans un mélange hexane-acétate d'éthyle. On obtient 4,3g (rdt : 65 %) de [(diphénylméthoxy) acétyl]-1 phényl-3 pipéridine. F = 102 - 103 ° C.

| Analyse centésimale : | | | |
|---|---|---|---|
| | C % | H % | N % |
| Calculé | 81,01 | 7,06 | 3,63 |
| Trouvé | 81,04 | 7,16 | 3,49 |

c) [(Diphénylméthoxy)-2 éthyl]-1 phényl-3 pipéridine, oxalate

Une solution de 3g (7,78 mmoles) de [(diphénylméthoxy)acétyl]-1 phényl-3 pipéridine dans 30 $cm^3$ de tétrahydrofuranne est ajoutée goutte à goutte à une solution de 0,5 g (12,4 mmoles) d'hydrure de lithium et d'aluminium dans 50 $cm^3$ de tétrahydrofuranne. Le mélange est porté 4 heures à reflux, abandonné une nuit au repos puis hydrolysé par addition de 2,5 $cm^3$ d'eau. On dilue avec 100$cm^3$ d'éther. Les boues alcalines sont séparées et lavées à l'éther. Les phases éthérées sont rassemblées, séchées sur sulfate de sodium et concentrées sous pression réduite. Le résidu est dissous dans 20 $cm^3$ d'acétone. On ajoute une solution de 0,75 g (8,3 mmoles) d'acide oxalique dans 30 $cm^3$ d'acétone. Le précipité d'oxalate de [-(diphénylméthoxy)-2 éthyl]-1 phényl-3 pipéridine est filtré puis recristallisé dans l'acétone. Rdt : 1,6 g (44,5 %). F = 90 - 92 °C.

| Analyse centésimale : $C_{28}H_{31}NO_5$ (M = 461,56) | | | |
|---|---|---|---|
| | C % | H % | N % |
| Calculé<br>Trouvé | 72,86<br>73,09 | 6,77<br>6,90 | 3,03<br>3,15 |

R.M.N. (DMSO $d_6$ + $CF_3COOD$)
$\delta$ = 1,4-2,1 (massif complexe),4H) ; 2,5-4,0 (massif complexe, 9H) ; 5,4 (singulet, 1H) ; 7,2 (singulet, 15H).

EXEMPLE 18

[(Diphénylméthoxy)-2 éthyl]-1 phényl-4 pipéridine, oxalate

a) [(Diphénylméthoxy) acétyl]-1 phényl-4 pipéridine, oxalate

En opérant comme dans l'exemple 8 à partir de 4,5 g (17,2 mmoles) de chlorure de (diphénylméthoxy) acétyl et de 2,8 g (17,2 mmoles) de phényl-4 pipéridine on obtient, après filtration sur silice (éluant hexane-acétate d'éthyle 1/1) et recristallisation dans un mélange hexane-acétate d'éthyle, 4,8 g (rdt : 72 %) de [-(diphénylméthoxy) acétyl]-1 phényl-4 pipéridine. F = 96 - 98 °C.

| Analyse centésimale : $C_{26}H_{27}NO_2$ | | | |
|---|---|---|---|
| | C % | H % | N % |
| Calculé<br>Trouvé | 81,01<br>80,81 | 7,06<br>7,06 | 3,63<br>3,60 |

b) [(Diphénylméthoxy)-2 éthyl]-1 phényl-4 pipéridine, oxalate

Une solution de 7,4 g (18,6 mmoles) de [(diphénylméthoxy) acétyl]-1 phényl-4 pipéridine dans 60 $cm^3$ de tétrahydrofuranne est ajoutée à une solution de 1,1 g (29,7 mmoles) d'hydrure de lithium et d'aluminium dans 100 $cm^3$ de tétrahydrofuranne. Le mélange est porté 12 heures à reflux puis, après retour à température ambiante, hydrolysé par 5,5 $cm^3$ d'eau. On ajoute 200 $cm^3$ d'éther. Les boues alcalines sont lavées à l'éther. Les solutions éthérées sont rassemblées, séchées sur sulfate de sodium et concentrées sous pression réduite. Le résidu est dissous dans 30 $cm^3$ d'acétone. On ajoute une solution de 1,7 g (18,9 mmoles) d'acide oxalique dans 150 $cm^3$ d'acétone. Le précipité d'oxalate de [(diphénylméthoxy)-2 éthyl]-1 phényl-4 pipéridine est filtré puis recristallisé dans un mélange acétone-éthanol.
Rdt : 5,1 g (59 %). F = 163 - 164 °C.

| Analyse centésimale : $C_{28}H_{31}NO_5$ (M = 461,56) | | | |
|---|---|---|---|
| | C % | H % | N % |
| Calculé | 72,86 | 6,77 | 3,03 |
| Trouvé | 72,70 | 6,78 | 3,09 |

EXEMPLE 19

[(Diphénylméthoxy)-2 éthyl]-1 (méthoxy-3 phényl)-3 pipéridine, oxalate

a) [(Diphénylméthoxy) acétyl]-1 (méthoxy-3 phényl)-3 pipéridine

Une solution de 2,8 g (10,9 mmoles) de chlorure de (diphénylméthoxy) acétyle dans 25 $cm^3$ de chlorure de méthylène est ajoutée rapidement à une solution de 2,1 g (10,9 mmoles) de(méthoxy-3 phényl)-3 pipéridine [obtenue selon la méthode de B.M. Iselin et K. Hoffmann, Helv. Chim. Acta 1954, 37, 178] et de 2,2 g (21,8 mmoles) de triéthylamine dans 75 $cm^3$ de chlorure de méthylène. La solution est portée à reflux pendant 10 heures puis versée dans une solution de 10 $cm^3$ d'acide chlorhydrique dans 300 $cm^3$ d'eau. Le mélange est extrait au chlorure de méthylène, lavé à l'eau et séché sur sulfate de sodium. Après évaporation du solvant sous pression réduite le résidu est purifié par chromatographie sur silice (éluant hexane-acétate d'éthyl 1/1). On obtient 3,3 g (rdt : 73 %)de [(diphénylméthoxy) acétyl]-1 (méthoxy-3 phényl)-3 pipéridine.

| Analyse centésimale : | | | |
|---|---|---|---|
| | C % | H % | N % |
| Calculé | 78,04 | 7,03 | 3,37 |
| Trouvé | 77,79 | 6,75 | 3,41 |

b) [(Diphénylméthoxy)-2 éthyl]-1 (méthoxy-3 phényl)-3 pipéridine, oxalate

Une solution de 6,6 g (15,9 mmoles) de [(diphénylméthoxy) acétyl]-1 (méthoxy -3 phényl)-3 pipéridine dans 50 $cm^3$ de tétrahydrofuranne est ajoutée goutte à goutte à une solution de 0,96 g (25,4 mmoles) d'hydrure de lithium et d'aluminium dans 100 $cm^3$ de tétrahydrofuranne. Le mélange est porté 5 heures à reflux, laissé au repos une nuit puis hydrolysé par addition de 5 $cm^3$ d'eau. Les boues alcalines sont séparées. La solution est concentrée sous pression réduite. Le résidu est traité par une solution de 1,5 g (16,7 mmoles) d'acide oxalique dans 100 $cm^3$ d'acétone. Le précipité d'oxalate de [(diphénylméthoxy)-2 éthyl]-1 (méthoxy-3 phényl)-3 pipéridine est filtré et recristallisé dans l'acétone. Rdt : 3,0 g (38 %). F = 80 - 86 °C.

| Analyse centésimale : $C_{29}H_{33}NO_6$ (M = 491,58) | | | |
|---|---|---|---|
| | C % | H % | N % |
| Calculé | 70,86 | 6,77 | 2,85 |
| Trouvé | 70,79 | 6,62 | 2,89 |

**Revendications**

**Revendications pour les Etats contractants suivants : AT, BE, CH, DE, FR, GB, IT, LI, LU, NL, SE**

1. [(Diarylméthoxy) alcoyl]-1 pyrrolidines et pipéridines caractérisées par la formule

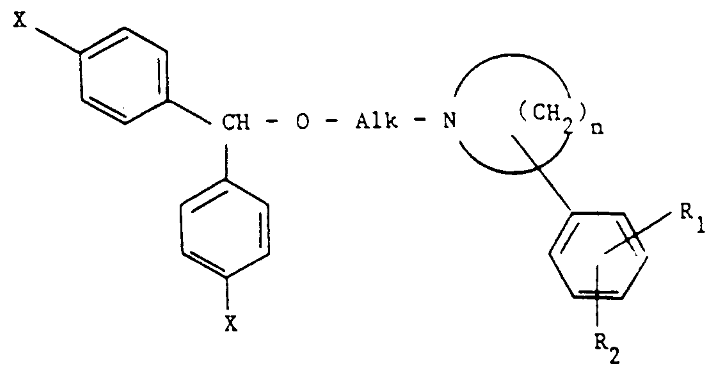

dans laquelle X est l'hydrogène ou un atome de fluor, Alk est un groupe acoyle linéaire ou ramifié contenant deux ou trois atomes de carbone ; $R_1$ et $R_2$ sont l'hydrogène ou un radical alcoxy linéaire ou ramifié comprenant de 1 à 4 atomes de carbones ; n est 4 ou 5 ; et leurs sels d'acides minéraux ou organiques phamaceutiquement acceptables.

2. [(Diarylméthoxy alcoyl]-1 pyrrolidines et pipéridines selon la revendication 1 caractérisée en ce que Alk est un radical linéaire contenant deux ou trois atomes de carbone ; et leurs sels d'acides minéraux ou organiques pharmaceutiquement acceptables

3. [(Diarylméthoxy) alcoyl]-1 pyrrolidines et pipéridines selon la revendication 1 caractérisées en ce que Alk est le radical éthylène $-CH_2CH_2-$ ; et leurs sels d'acides minéraux ou organiques pharmaceutiquement acceptables.

4. [(Diarylméthoxy) alcoyl]-1 pyrrolidines selon la revendication 1, de formule générale

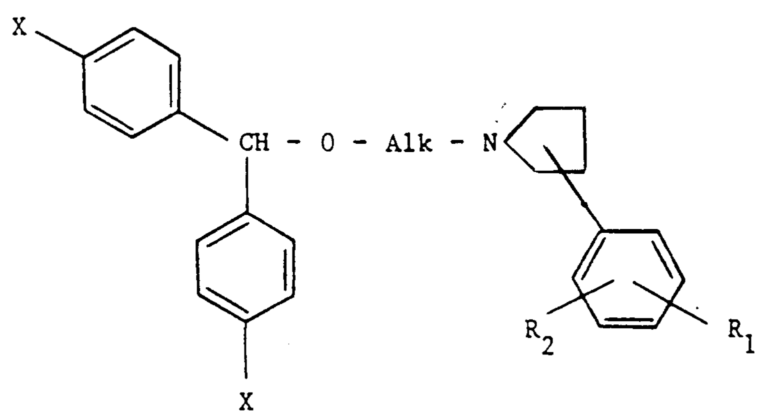

dans laquelle X, Alk, $R_1$ et $R_2$ sont tels que définis à la revendication 1, et leurs sels d'acides minéraux ou organiques pharmaceutiquement acceptables.

5. [(Diarylméthoxy) alcoyl]-1 pyrrolidines selon la revendication 4, caractérisées en ce que X représente un atome de fluor, et leurs sels d'acides minéraux ou organiques pharmaceutiquement acceptables

6. [(Diarylméthoxy) alcoyl]-1 pipéridines selon la revendication 1, de formule générale

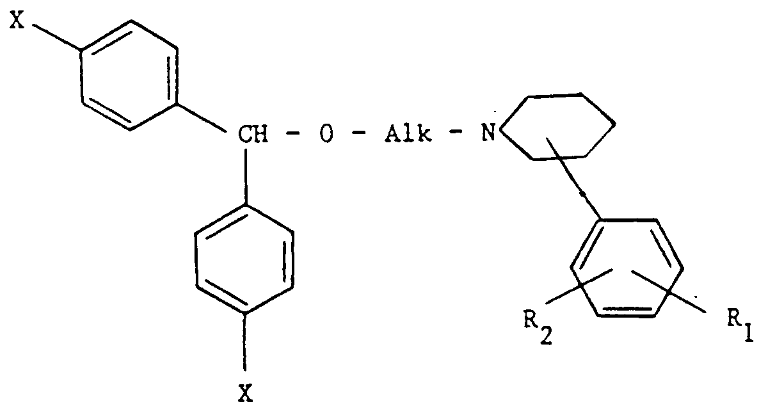

dans laquelle X, Alk, $R_1$ et $R_2$ sont tels que définis à la revendication 1, et leurs sels d'acides minéraux ou organiques pharmaceutiquement acceptables

7. [(Diarylméthoxy) alcoyl]-1 pipéridines selon la revendication 6, caractérisées en ce que X représente un atome de fluor, et leurs sels d'acides minéraux ou organiques phamaceutiquement acceptables.

8. [(Diarylméthoxy) alcoyl]-1 pyrrolidines selon la revendication 1 caractérisées en ce que Alk est le radical éthylène $-CH_2CH_2-$ et n est 4 ; et leurs sels d'acides minéraux ou organiques pharmaceutiquement acceptables.

9. Le composé [(diphénylméthoxy)-2 éthyl]-1 (méthoxy-3 phényl)-3 pyrrolidine ; et ses sels d'acides minéraux ou organiques pharmaceutiquement acceptables.

10. Le composé [[bis (fluoro-4 phényl) méthoxy]-2 éthyl]-1 (méthoxy-3 phényl)-3 pyrrolidine ; et ses sels d'acides minéraux ou organiques pharmaceutiquement acceptables.

11. Procédé de préparation des composés selon la revendication 1 caractérisé en ce qu'on condense un dérivé de formule

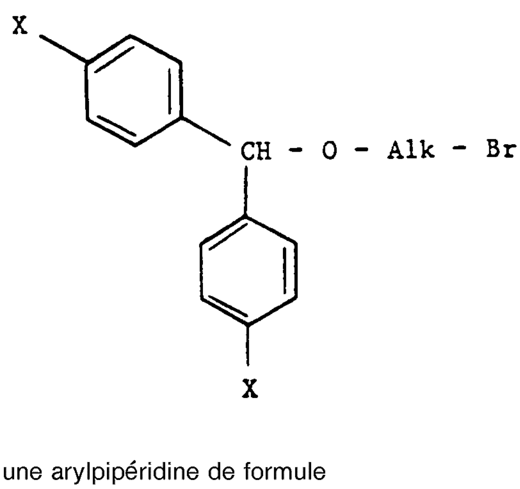

avec une arylpyrrolidine ou une arylpipéridine de formule

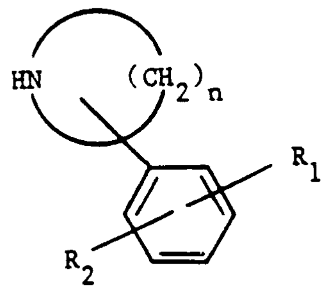

en présence d'un agent alcalin,
X, Alk, $R_1$, $R_2$ et n ayant les significations données dans la revendication 1.

**12.** Procédé de préparation des composés selon la revendication 1 pour lesquels Alk est $-CH_2CH_2-$ et X = H caractérisé en ce qu'on condense un chlorure d'acide de formule

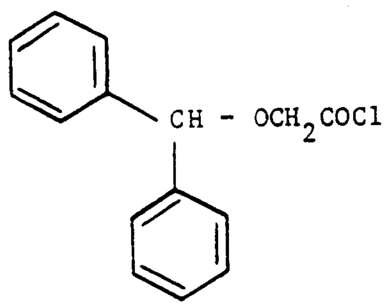

avec une arylpyrrolidine ou une arylpipéridine de formule

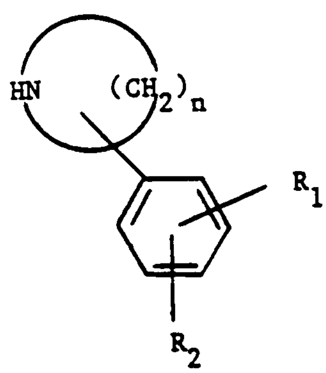

pour conduire à un amide formule

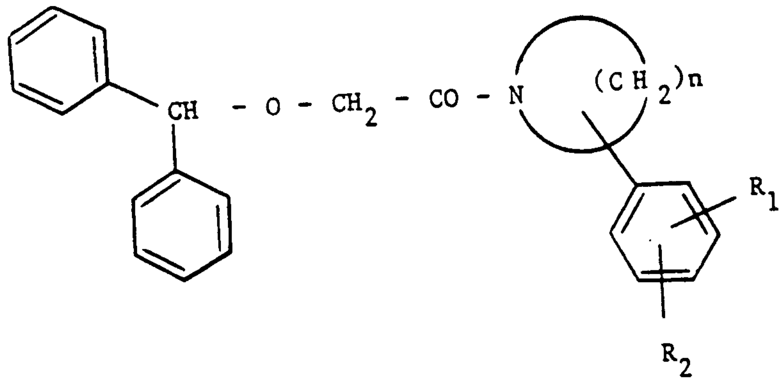

qui est réduit par l'hydrure de lithium et d'aluminium, $R_1$, $R_2$ et n ayant les significations données dans la revendications 1.

**13.** Médicament contenant comme principe actif une [(diarylméthoxy) alcoyl]-1 pyrrolidine ou pipéridine caractérisée par la formule

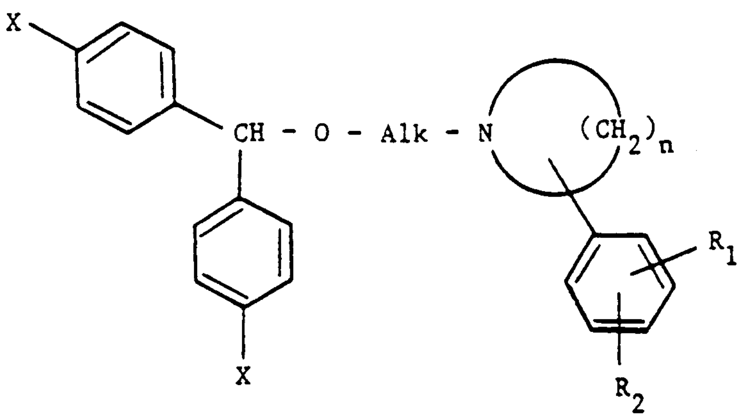

dans laquelle X est l'hydrogène ou un atome de fluor, Alk est un groupe alcoyle linéaire ou ramifié contenant deux ou trois atomes de carbone ; $R_1$ er $R_2$ sont l'hydrogène ou un radical alcoxy linéaire ou ramifié comprenant de 1 à 4 atomes de carbone ; n est 4 ou 5 ; et ses sels d'acides minéraux ou organiques phamaceutiquement acceptables.

**Revendications pour l'Etat contractant suivant : ES**

**1.** Procédé de préparation de [(diarylméthoxy) alcoyl]-1 pyrrolidines et pipéridines caractérisées par la formule

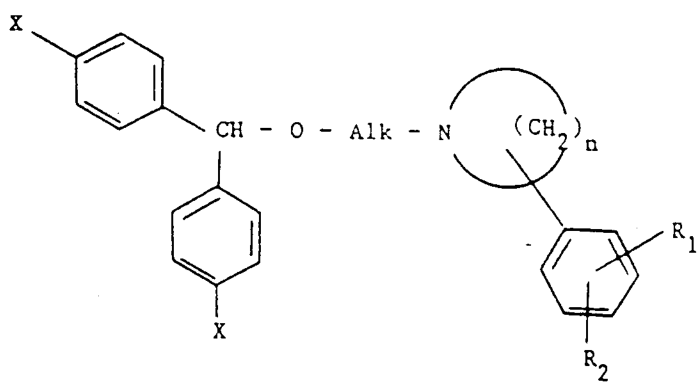

dans laquelle X est l'hydrogène ou un atome de fluor, Alk est un groupe acoyle linéaire ou ramifié contenant deux ou trois atomes de carbone ; $R_1$ et $R_2$ sont l'hydrogène ou un radical alcoxy linéaire ou ramifié comprenant de 1 à 4 atomes de carbones ; n est 4 ou 5 ; et leurs sels d'acides minéraux ou organiques phamaceutiquement acceptables, caractérisé en ce qu'on condense un dérivé de formule

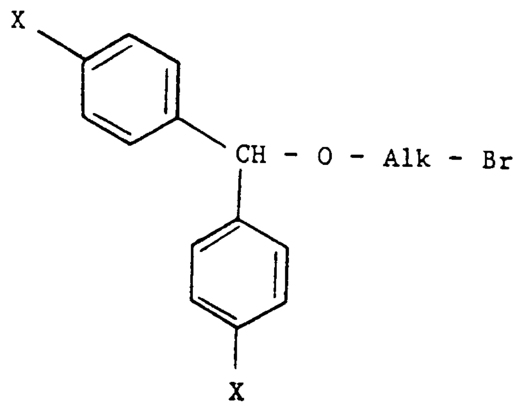

avec une arylpyrrolidine ou une arylpipéridine de formule

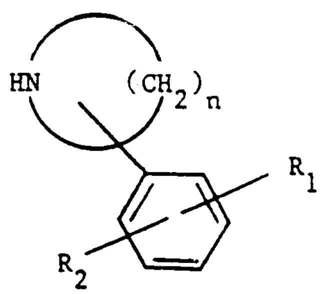

en présence d'un agent alcalin,
X, Alk, $R_1$, $R_2$ et n ayant les significations données ci-dessus.

**2.** Procédé de préparation des composés selon la revendication 1 pour lesquels Alk est $-CH_2CH_2-$ et X = H caractérisé en ce qu'on condense un chlorure d'acide de formule

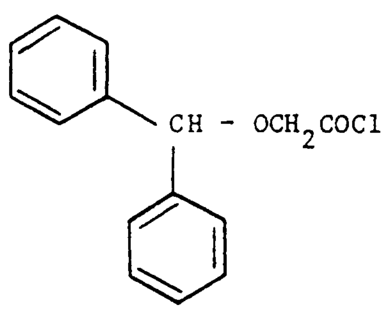

avec une arylpyrrolidine ou une arylpipéridine de formule

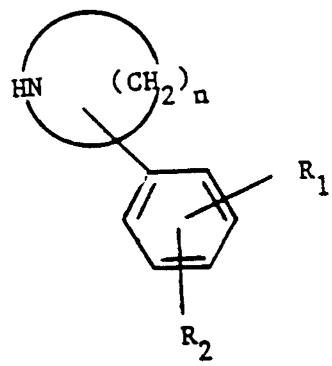

pour conduire à un amide formule

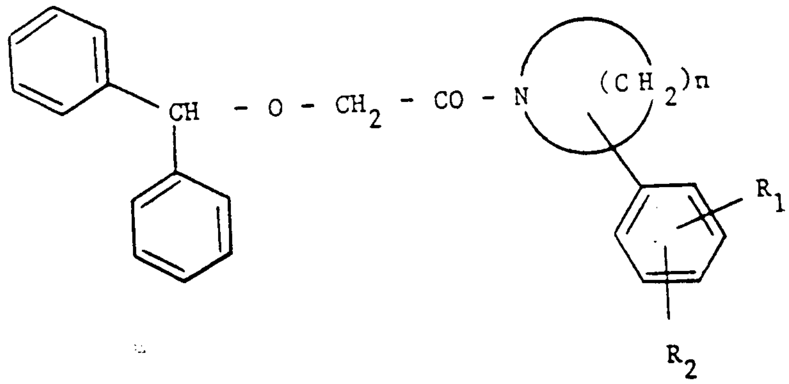

qui est réduit par l'hydrure de lithium et d'aluminium, $R_1$, $R_2$ et n ayant les significations données dans la revendication 1

3. Procédé selon la revendication 1 caractérisé en ce qu'on prépare des [(diarylméthoxy) alcoyl]-1 pyrrolidines et pipéridines dans lesquels Alk est un radical linéaire contenant deux ou trois atomes de carbone ; et leurs sels d'acides minéraux ou organiques pharmaceutiquement acceptables.

4. Procédé selon la revendication 1, caractérisé en ce qu'on prépare des [(diarylméthoxy)alcoyl]-1 pyrrolidines et pipéridines dans lesquels Alk est le radical éthylène $-CH_2CH_2-$ ; et leurs sels d'acides minéraux ou organiques pharmaceutiquement acceptables.

5. Procédé selon la revendication 1 caractérisé en qu'on prépare des [(diarylméthoxy) alcoyl]-1pyrrolidines, de formule générale

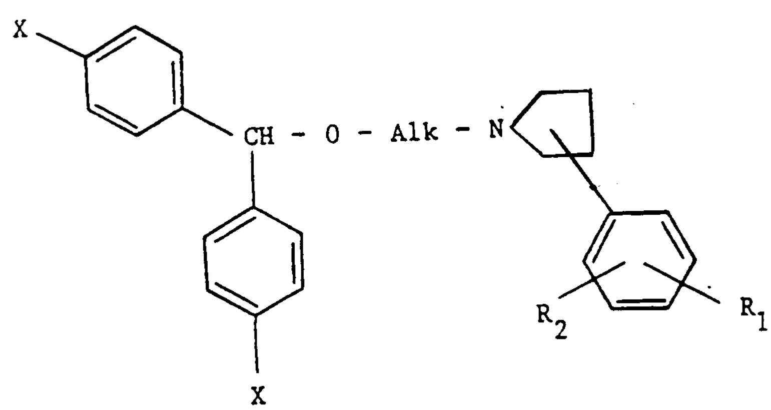

dans laquelle X, Alk, $R_1$ et $R_2$ sont tels que définis à la revendication 1, et leurs sels d'acides minéraux ou organiques pharmaceutiquement acceptables.

6. Procédé selon la revendication 5, caractérisé en ce que X représente un atome de fluor.

7. Procédé selon la revendication 1 caractérisé en ce qu'on prépare des [(diarylméthoxy) alcoyl]-1 pipéridines de formule générale

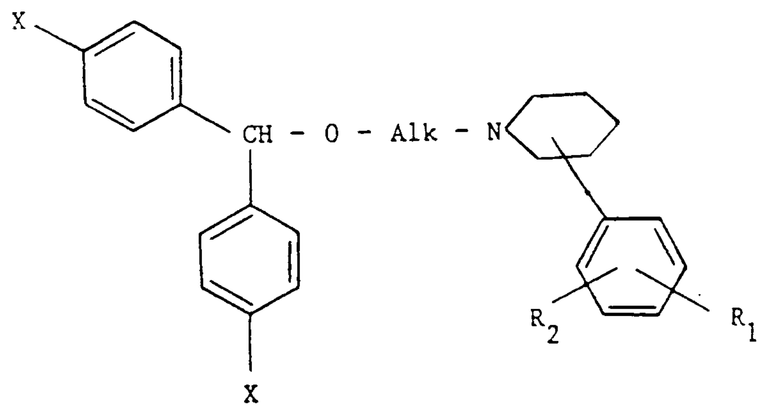

dans laquelle X, Alk, $R_1$ et $R_2$ sont tels que définis à la revendication 1, et leurs sels d'acides minéraux ou organiques pharmaceutiquement acceptables

8. Procédé selon la revendication 7 caractérisé en ce que X représente un atome de fluor.

9. Procédé selon la revendication 1 caractérisé en ce que l'on prépare des [(diarylméthoxy) alcoyl)-1 pyrrolidines dans lesquelles Alk est le radical éthylène $-CH_2CH_2-$ et n est 4 ; et leurs sels d'acides minéraux ou organiques pharmaceutiquement acceptables.

10. Procédé selon la revendication 1 caractérisé en ce qu'on prépare la [(diphénylméthoxy)-2 éthyl]-1 (méthoxy-3 phényl)-3 pyrrolidine par condensation du (diphénylméthoxy)-1 bromo-2 éthane avec la (méthoxy-3 phényl)-3 pyrrolidine.

11. Procédé selon la revendication 1, caractérisé en ce que l'on prépare la [[bis (fluoro-4 phényl) méthoxy]-2 éthyl]-1 (méthoxy-3 phényl)-3 pyrrolidine par condensation du (bis (fluoro-4 phényl) méthoxy)-1 bromoéthane avec la (méthoxy-3 phényl)-3 pyrrolidine en présence d'hydrure de sodium.

**Revendications pour l'Etat contractant suivant : GR**

1. [(Diarylméthoxy) alcoyl]-1 pyrrolidines et pipéridines caractérisées par la formule

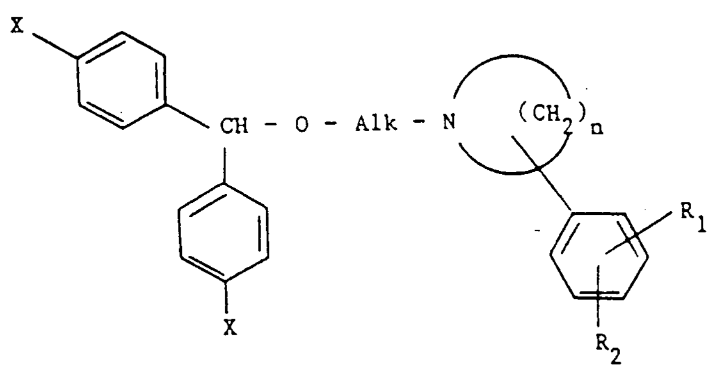

dans laquelle X est l'hydrogène ou un atome de fluor, Alk est un groupe acoyle linéaire ou ramifié

contenant deux ou trois atomes de carbone ; $R_1$ et $R_2$ sont l'hydrogène ou un radical alcoxy linéaire ou ramifié comprenant de 1 à 4 atomes de carbones ; n est 4 ou 5 ; et leurs sels d'acides minéraux ou organiques phamaceutiquement acceptables.

**2.** [(Diarylméthoxy) alcoyl]-1 pyrrolidines et pipéridines selon la revendication 1 caractérisée en ce que Alk est un radical linéaire contenant deux ou trois atomes de carbone ; et leurs sels d'acides minéraux ou organiques pharmaceutiquement acceptables.

**3.** [(Diarylméthoxy) alcoyl]-1 pyrrolidines et pipéridines selon la revendication 1 caractérisées en ce que Alk est le radical éthylène $-CH_2CH_2-$ ; et leurs sels d'acides minéraux ou organiques pharmaceutiquement acceptables.

**4.** [(Diarylméthoxy) alcoyl]-1 pyrrolidines selon la revendication 1, de formule générale

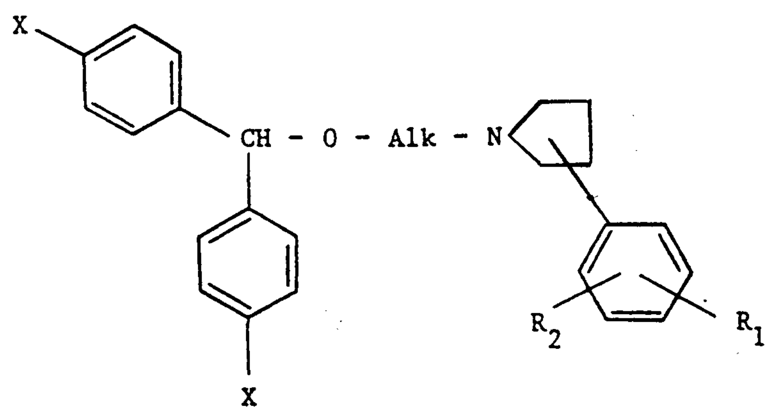

dans laquelle X, Alk, $R_1$ et $R_2$ sont tels que définis à la revendication 1, et leurs sels d'acides minéraux ou organiques pharmaceutiquement acceptables.

**5.** [(Diarylméthoxy) alcoyl]-1 pyrrolidines selon la revendication 4, caractérisées en ce que X représente un atome de fluor, et leurs sels d'acides minéraux ou organiques pharmaceutiquement acceptables

**6.** [(Diarylméthoxy) alcoyl]-1 pipéridines selon la revendication 1, de formule générale

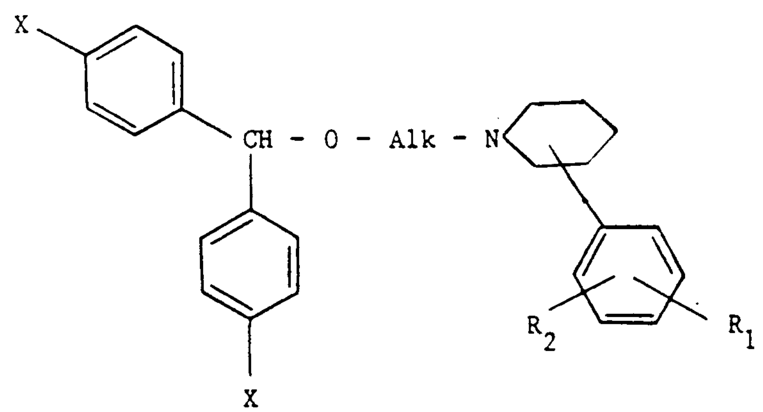

dans laquelle X, Alk, $R_1$ et $R_2$ sont tels que définis à la revendication 1, et leurs sels d'acides minéraux ou organiques pharmaceutiquement acceptables

**7.** [(Diarylméthoxy) alcoyl]-1 pipéridines selon la revendication 6, caractérisées en ce que X représente un atome de fluor, et leurs sels d'acides minéraux ou organiques phamaceutiquement acceptables.

**8.** [(Diarylméthoxy) alcoyl]-1 pyrrolidines selon la revendication 1 caractérisées en ce que Alk est le radical éthylène $-CH_2CH_2-$ et n est 4 ; et leurs sels d'acides minéraux ou organiques pharmaceutiquement acceptables.

**9.** Le composé [(diphénylméthoxy)-2 éthyl]-1 (méthoxy-3 phényl)-3 pyrrolidine ; et ses sels d'acides minéraux ou organiques pharmaceutiquement acceptables.

**10.** Le composé [[bis (fluoro-4 phényl) méthoxy]-2 éthyl]-1 (méthoxy-3 phényl)-3 pyrrolidine ; et ses sels d'acides minéraux ou organiques pharmaceutiquement acceptables.

**11.** Procédé de préparation des composés selon la revendication 1 caractérisé en ce qu'on condense un dérivé de formule

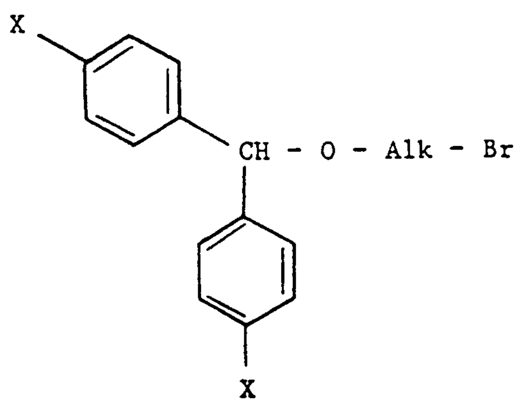

avec une arylpyrrolidine ou une arylpipéridine de formule

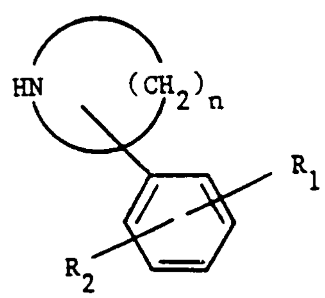

en présence d'un agent alcalin,
X, Alk, $R_1$, $R_2$ et n ayant les significations données dans la revendication 1.

**12.** Procédé de préparation des composés selon la revendication 1 pour lesquels Alk est $-CH_2CH_2-$ et X = H caractérisé en ce qu'on condense un chlorure d'acide de formule

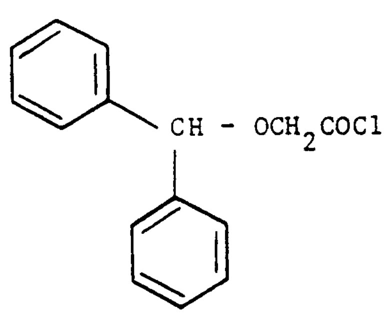

avec une arylpyrrolidine ou une arylpipéridine de formule

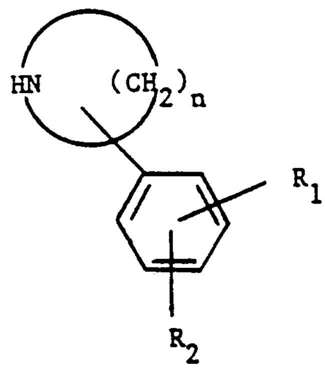

pour conduire à un amide formule

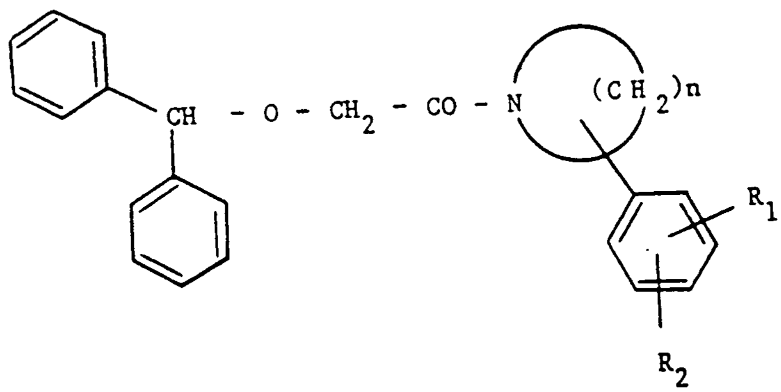

qui est réduit par l'hydrure de lithium et d'aluminium, $R_1$, $R_2$ et n ayant les significations données dans la revendications 1.

## Claims

**Claims for the following Contracting States : AT, BE, CH, DE, FR, GB, IT, LI, LU, NL, SE**

**1.** [(Diarylmethoxy)-alkyl]-1-pyrrolidines and piperidines, characterized by the formula

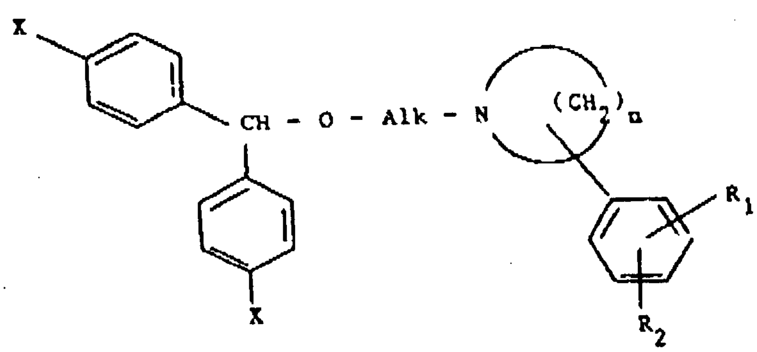

in which X is hydrogen or a fluorine atom, Alk is a straight or branched alkyl group containing two or three carbon atoms, $R_1$ and $R_2$ are hydrogen or a straight or branched alkoxy radical having 1 to 4 carbon atoms, n is 4 or 5, and their salts of pharmaceutically acceptable mineral or organic acids.

**2.** [(Diarylmethoxy)-alkyl]-1-pyrrolidines and piperidines according to claim 1, characterized in that Alk is a straight radical containing two or three carbon atoms, and their salts of pharmaceutically acceptable mineral or organic acids.

**3.** [Diarylmethoxy)-alkyl]-1-pyrrolidines and piperidines according to claim 1, characterized in that Alk is the ethylene radical $-CH_2CH_2$, and their salts of pharmaceutically acceptable mineral or organic acids.

**4.** [(Diarylmethoxy)-alkyl]-1-pyrrolidines according to claim 1 of general formula

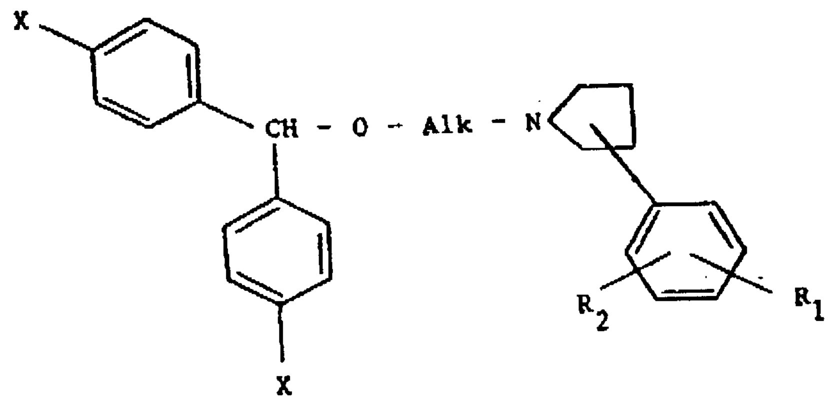

in which X, Alk, $R_1$ and $R_2$ are as defined in claim 1, and their salts of pharmaceutically acceptable mineral or organic acids.

**5.** [(Diarylmethoxy)-alkyl]-1-pyrrolidines according to claim 4, characterized in that X represents a fluorine atom, and their salts of pharmaceutically acceptable mineral or organic acids.

**6.** [(Diarylmethoxy)-alkyl]-1-piperidines according to claim 1 of general formula

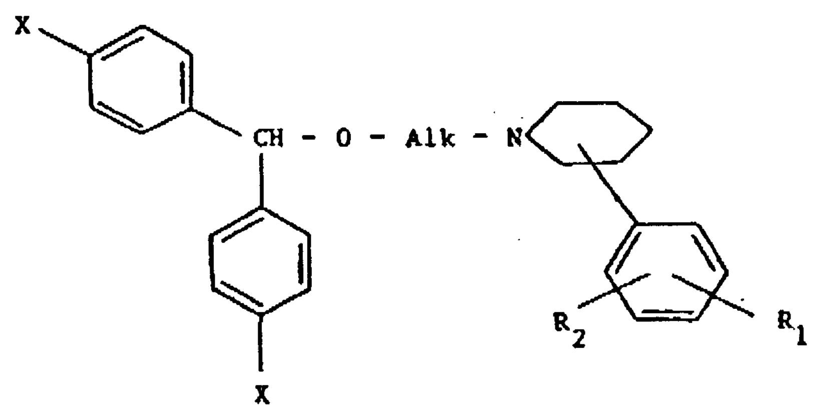

in which X, Alk, $R_1$ and $R_2$ are as defined in claim 1, and their salts of pharmaceutically acceptable mineral or organic acids.

**7.** [(Diarylmethoxy)-alkyl]-1-piperidines according to claim 6, characterized in that X represents a fluorine atom and their salts of pharmaceutically acceptable mineral or organic acids.

**8.** [(Diarylmethoxy)-alkyl]-1-pyrrolidines according to claim 1, characterized in that Alk is the ethylene radical $-CH_2CH_2-$ and n is 4 and their salts of pharmaceutically acceptable mineral or organic acids.

**9.** The compound [(diphenylmethoxy)-2-ethyl]-1-(3-methoxyphenyl)-3-pyrrolidine, and their salts of pharmaceutically acceptable mineral or organic acids.

**10.** The compound [[bis (4-fluorophenyl)-methoxy]-2-ethyl]-1-(3-methoxyphenyl)-3-pyrrolidine, and their salts of pharmaceutically acceptable mineral or organic acids.

**11.** Process for the preparation of compounds according to claim 1, characterized in that condensation takes place of a derivative of formula

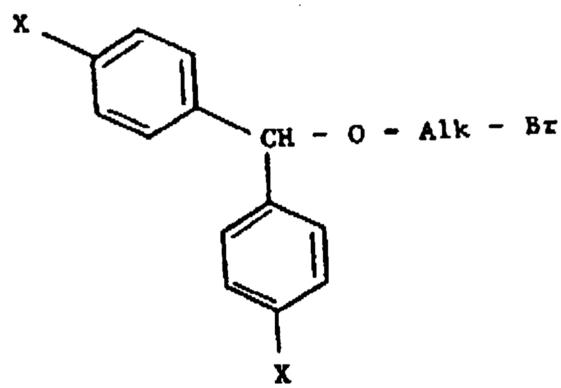

with an aryl pyrrolidine or an aryl piperidine of formula

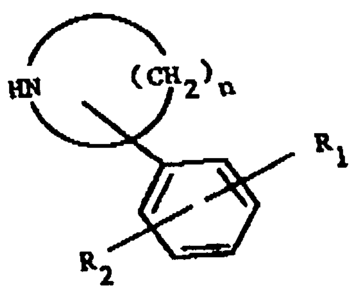

in the presence of an alkaline agent, X, Alk, $R_1$, $R_2$ and n having the meanings given in claim 1.

**12.** Process for the preparation of compounds according to claim 1 for which Alk is $-CH_2CH_2-$ and X = H, characterized in that condensation takes place of an acid chloride of formula

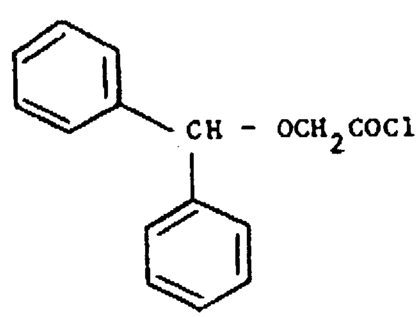

with an aryl pyrrolidine or an aryl piperidine of formula

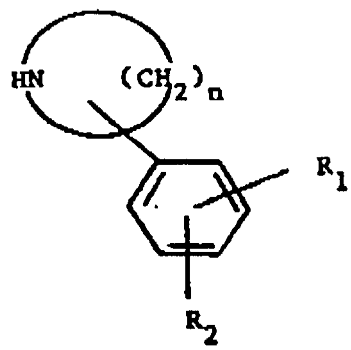

in order to lead to an amide of formula

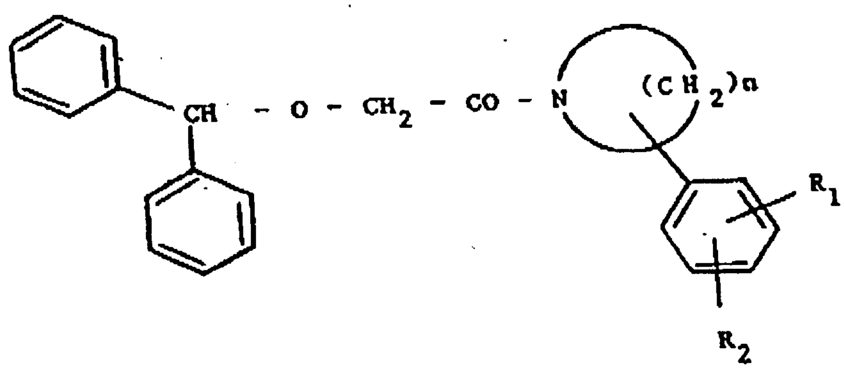

which is reduced by aluminium and lithium hydride, $R_1$, $R_2$ and n having the meanings given in claim 1.

**13.** Medicament containing as the active principle a [(diarylmethoxy)-alkyl]-1-pyrrolidine or piperidine, characterized by the formula

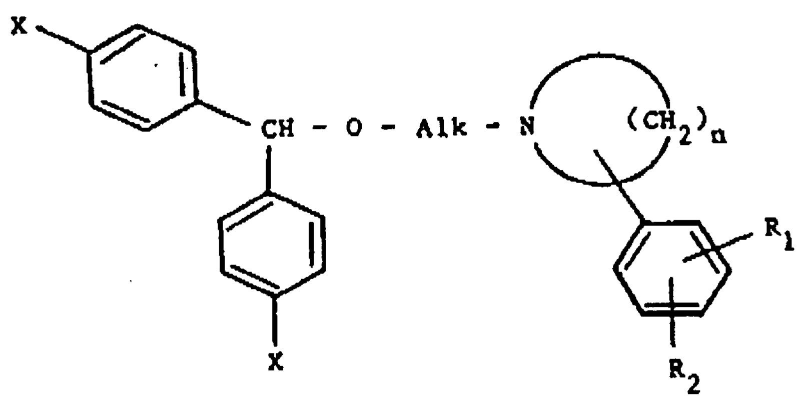

in which X is hydrogen or a fluorine atom, Alk is a straight or branched alkyl group containing two or three carbon atoms, $R_1$ and $R_2$ are hydrogen or a straight or branched alkoxy radical having 1 to 4 carbon atoms, n is 4 or 5, and the salts of pharmaceutically acceptable mineral or organic acids.

**Claims for the following Contracting State : ES**

**1.** Process for the preparation of [(Diarylmethoxy)-alkyl]-1-pyrrolidines and piperidines, characterized by the formula

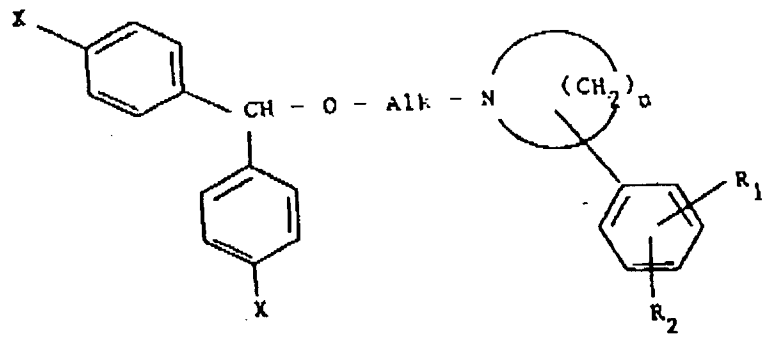

in which X is hydrogen or a fluorine atom, Alk is a straight or branched alkyl group containing two or three carbon atoms, $R_1$ and $R_2$ are hydrogen or a straight or branched alkoxy radical having 1 to 4 carbon atoms, n is 4 or 5, and their salts of pharmaceutically acceptable mineral or organic acids, characterized in that condensation takes place of a derivative of formula

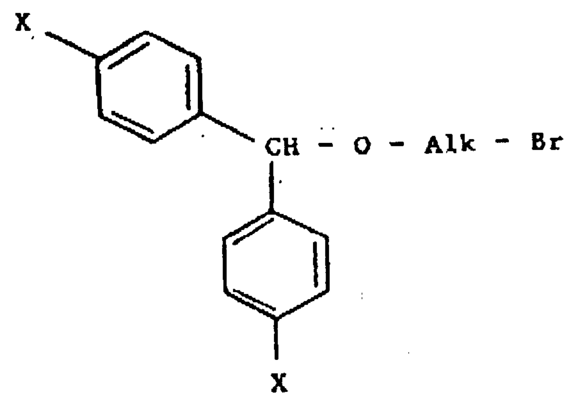

with an aryl pyrrolidine or an aryl piperidine of formula

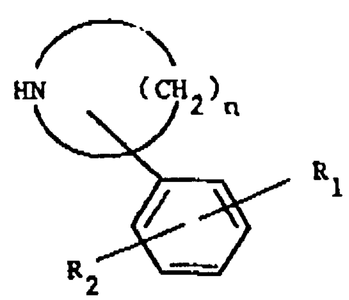

in the presence of an alkaline agent, X, Alk, $R_1$, $R_2$ and n having the meanings given hereinbefore.

**2.** Process for the preparation of compounds according to claim 1 for which Alk is $-CH_2CH_2-$ and X = H, characterized in that condensation takes place of an acid chloride of formula

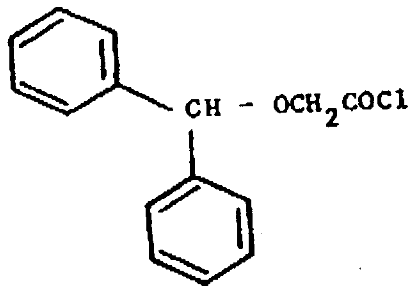

with an aryl pyrrolidine or an aryl piperidine of formula

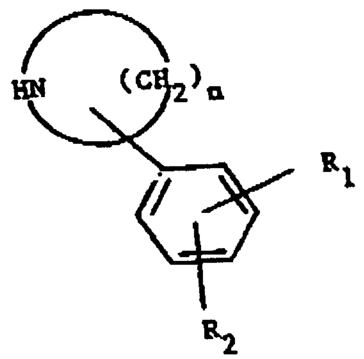

in order to lead to an amide of formula

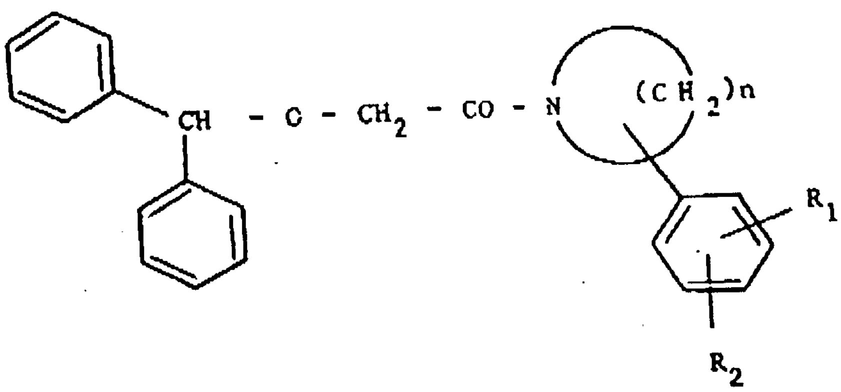

which is reduced by aluminium and lithium hydride, $R_1$, $R_2$ and n having the meanings given in claim 1.

3. Process according to claim 1, characterized in that [(diarylmethoxy)-alkyl]-1-pyrrolidines and piperidines are prepared, in which Alk is a straight radical containing two or three carbon atoms, and their salts of pharmaceutically acceptable mineral or organic acids.

4. Process according to claim 1, characterized in that [(diarylmethoxy)-alkyl]-1-pyrrolidines and piperidines are prepared, in which Alk is the ethylene radical $-CH_2CH_2-$, and their salts of pharmaceutically acceptable mineral or organic acids.

5. Process according to claim 1, characterized in that [(diarylmethoxy)-alkyl]-1-pyrrolidines are prepared of general formula

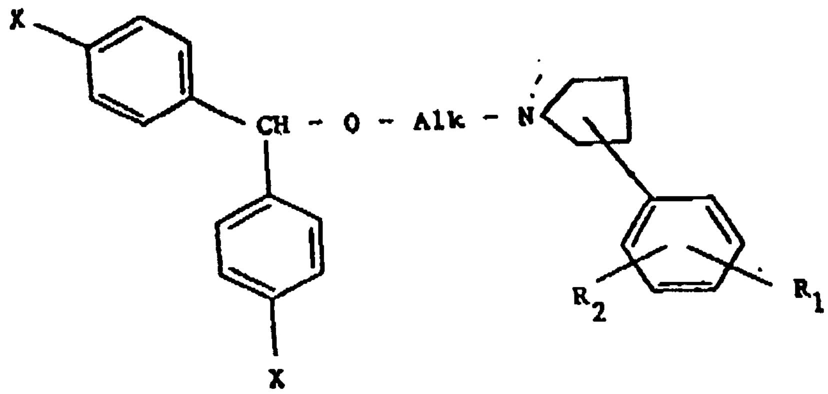

in which X, Alk, $R_1$ and $R_2$ are as defined in claim 1, and their salts of pharmaceutically acceptable mineral or organic acids.

**6.** Process according to claim 5, characterized in that X represents a fluorine atom.

**7.** Process according to claim 1, characterized in that [(diarylmethoxy)-alkyl]-1-piperidines are prepared of general formula

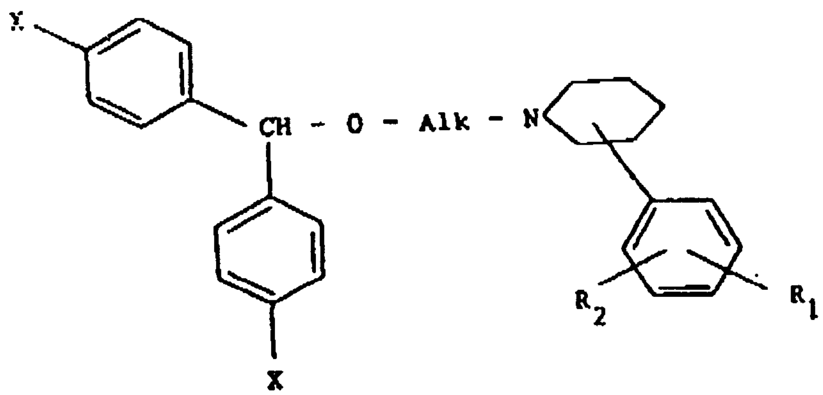

in which X, Alk, $R_1$ and $R_2$ are as defined in claim 1 and their salts of pharmaceutically acceptable mineral or organic acids.

**8.** Process according to claim 7, characterized in that X represents a fluorine atom.

**9.** Process according to claim 1, characterized in that [(diarylmethoxy)-alkyl]-1-pyrrolidines are prepared in which Alk is the ethylene radical $-CH_2CH_2-$ and n is 4, and their salts of pharmaceutically acceptable mineral or organic acids.

**10.** Process according to claim 1, characterized in that [(diphenylmethoxy)-2-ethyl]-1-(3-methoxyphenyl)-3-pyrrolidine is prepared by the condensation of 1-(diphenylmethoxy)-2-bromoethane with 3-(3-methoxyphenyl)-pyrrolidine.

**11.** Process according to claim 1, characterized in that [[bis (4-fluorophenyl)-methoxy]-2-ethyl]-1-(3-methoxyphenyl)-3-pyrrolidine by the condensation of ((4-fluorophenyl)-methoxy)-1-bromoethane with 3-(3-methoxyphenyl)-pyrrolidine in the presence of sodium hydride.

**Claims for the following Contracting State : GR**

1. [(Diarylmethoxy)-alkyl]-1-pyrrolidines and piperidines, characterized by the formula

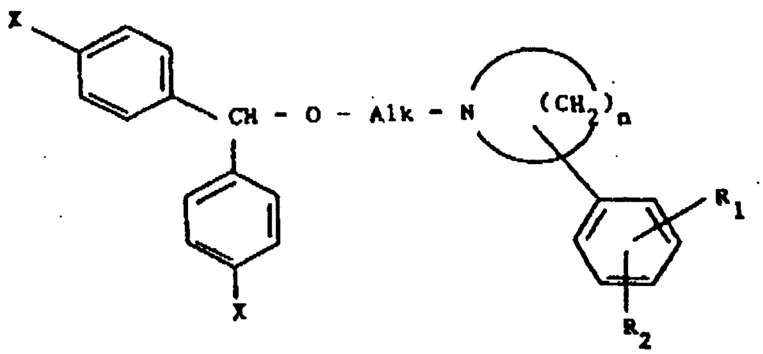

in which X is hydrogen or a fluorine atom, Alk is a straight or branched alkyl group containing two or three carbon atoms, $R_1$ and $R_2$ are hydrogen or a straight or branched alkoxy radical having 1 to 4 carbon atoms, n is 4 or 5, and their salts of pharmaceutically acceptable mineral or organic acids.

2. [(Diarylmethoxy)-alkyl]-1-pyrrolidines and piperidines according to claim 1, characterized in that Alk is a straight radical containing 2 or 3 carbon atoms and their salts of pharmaceutically acceptable mineral or organic acids.

3. [(Diarylmethoxy)-alkyl]-1-pyrrolidines and piperidines according to claim 1, characterized in that Alk is the ethylene radical $-CH_2CH_2-$, and their salts of pharmaceutically acceptable mineral or organic acids.

4. [(Diarylmethoxy)-alkyl]-1-pyrrolidines according to claim 1 of general formula

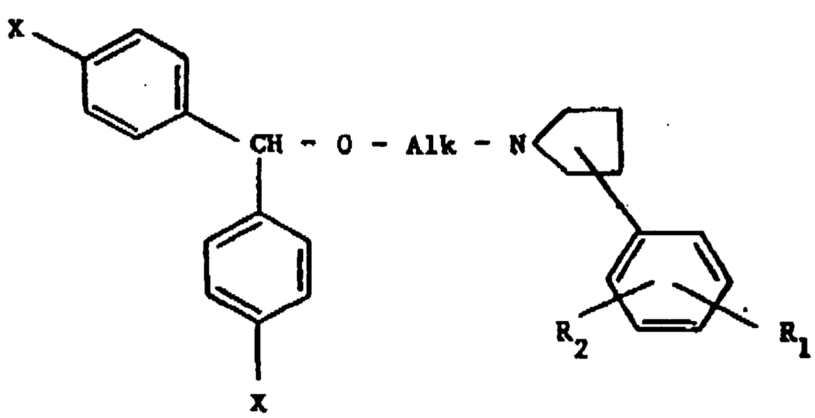

in which X, Alk, $R_1$ and $R_2$ are as defined in claim 1 and their salts of pharmaceutically acceptable mineral or organic acids.

5. [Diarylmethoxy-alkyl]-1-pyrrolidines according to claim 4, characterized in that X represents a fluorine atom, and their salts of pharmaceutically acceptable mineral or organic acids.

**6.** [(Diarylmethoxy-alkyl]-1-piperidines according to claim 1 of general formula

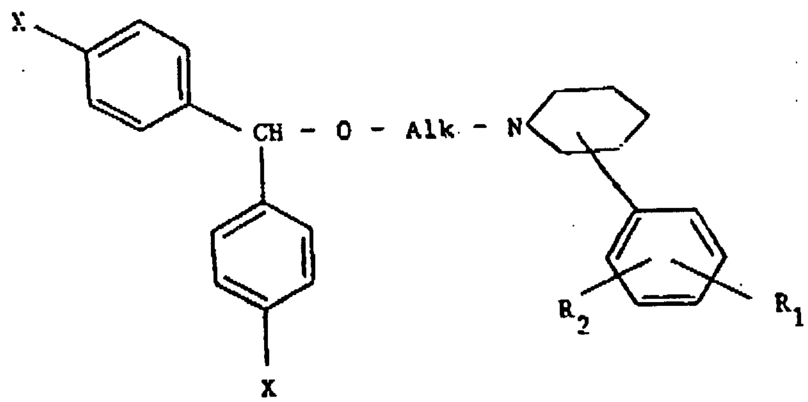

in which X, Alk, $R_1$ and $R_2$ are as defined in claim 1 and their salts of pharmaceutically acceptable mineral or organic acids.

**7.** [(Diarylmethoxy)-alkyl]-1-piperidines according to claim 6, characterized in that X represents a fluorine atom and their salts of pharmaceutically acceptable mineral or organic acids.

**8.** [(Diarylmethoxy)-alkyl]-1-pyrrolidines according to claim 1, characterized in that Alk is the ethylene radical $-CH_2CH_2-$ and n is 4, and their salts of pharmaceutically acceptable mineral or organic acids.

**9.** The compound [(diphenylmethoxy)-2-ethyl]-1-(3-methoxyphenyl)-3-pyrrolidine, and their salts of pharmaceutically acceptable mineral or organic acids.

**10.** The compound [[bis (4-fluorophenyl)-methoxy]-2-ethyl]-1-(3-methoxyphenyl)-3-pyrrolidine and their salts of pharmaceutically acceptable mineral or organic acids.

**11.** Process for the preparation of compounds according to claim 1, characterized in that condensation takes place of a derivative of formula

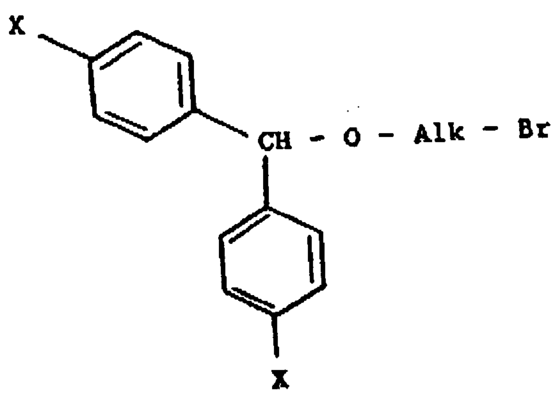

with an aryl pyrrolidine or an aryl piperidine of formula

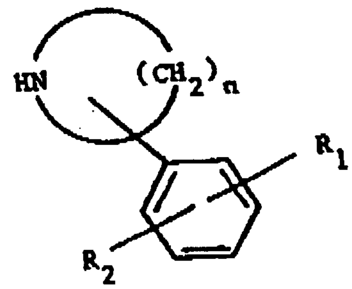

in the presence of an alkaline agent, X, Alk, $R_1$, $R_2$ and n having the meanings given in claim 1.

**12.** Process for the preparation of compounds according to claim 1 for which Alk is $-CH_2CH_2-$ and X = H, characterized in that condensation takes place of an acid chloride of formula

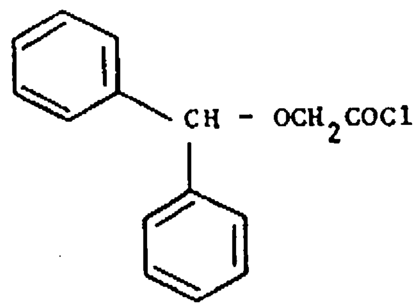

with an aryl pyrrolidine or an aryl piperidine of formula

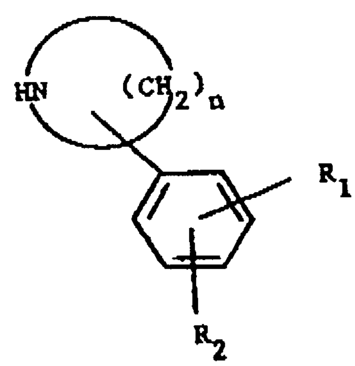

in order to lead to an amide of formula

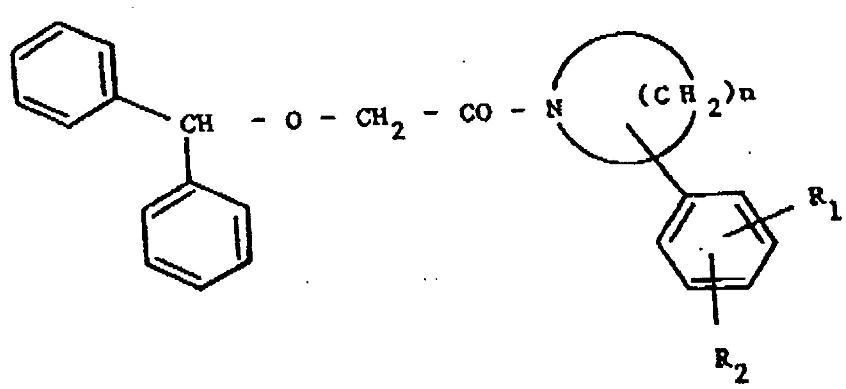

which is reduced by aluminium and lithium hydride, $R_1$, $R_2$ and n having the meanings given in claim 1.

**Patentansprüche**
**Patentansprüche für folgende Vertragsstaaten : AT, BE, CH, DE, FR, GB, IT, LI, LU, NL, SE,**

1. 1-[(Diarylmethoxy)alkyl] pyrrolidine und -piperidine, gekennzeichnet durch die Formel

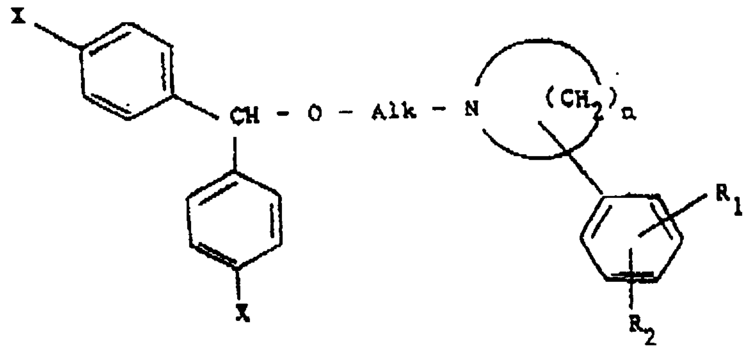

in der X Wasserstoff oder ein Fluoratom ist, Alk eine geradkettige oder verzweigte Alkylgruppe mit 2 oder 3 Kohlenstoffatomen ist; $R_1$ und $R_2$ Wasserstoff oder ein geradkettiger oder verzweigter Alkoxyrest mit 1 bis 4 Kohlenstoffatomen sind; n 4 oder 5 ist und ihre Mineralsäuresalze oder organischen, pharmazeutisch verträglichen Salze.

2. 1-[(Diarylmethoxy)alkyl] pyrrolidine und -piperidine nach Anspruch 1, dadurch gekennzeichnet, daß Alk ein geradkettiger, 2 oder 3 Kohlenstoffatome enthaltender Rest ist, und ihre Mineralsäuresalze oder organischen, pharmazeutisch verträglichen Salze.

3. 1-[(Diarylmethoxy)alkyl] pyrrolidine und -piperidine gemäß Anspruch 1, dadurch gekennzeichnet, daß Alk der Ethylenrest $-CH_2CH_2-$ ist, und ihre Mineralsäuresalze oder organischen, pharmazeutisch verträglichen Salze.

4. 1-[(Diarylmethoxy)alkyl] pyrrolidine gemäß Anspruch 1 der allgemeinen Formel

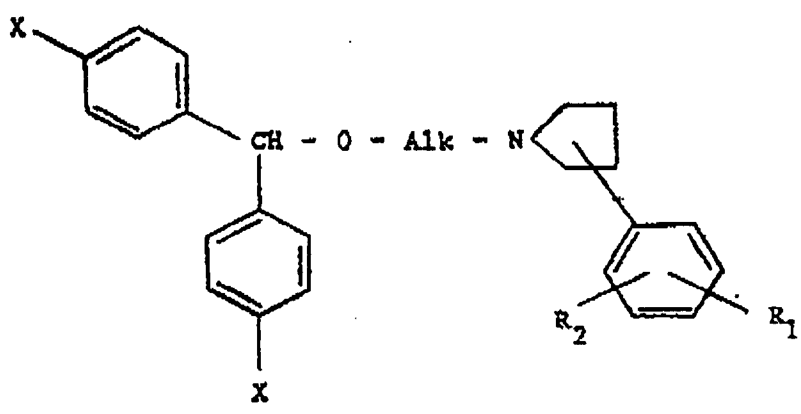

in der X, Alk, $R_1$ und $R_2$ die in Anspruch 1 angegebene Bedeutung haben, und ihre Mineralsäuresalze oder ihre organischen, pharmazeutisch verträglichen Salze.

5. 1-[(Diarylmethoxy)alkyl] pyrrolidine gemäß Anspruch 4, dadurch gekennzeichnet, daß X ein Fluoratom bedeutet, und ihre Mineralsäuresalze oder organischen, pharmazeutisch verträglichen Salze.

6. 1-[(Diarylmethoxy)alkyl] piperidine gemäß Anspruch 1 dar allgemeinen Formel

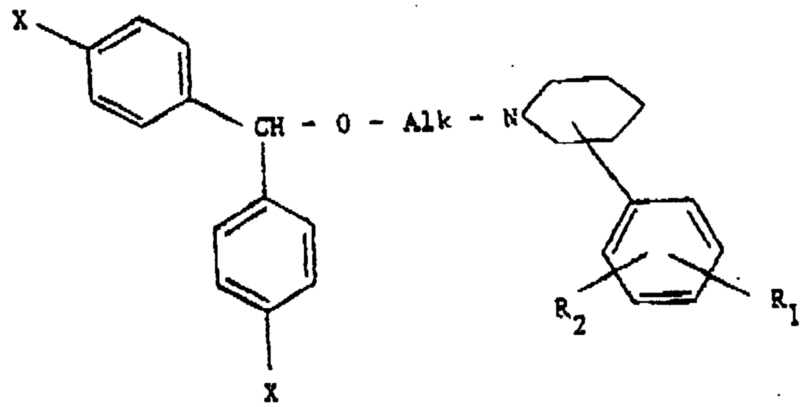

in der X, Alk, $R_1$ und $R_2$ dieselbe Bedeutung wie in Anspruch 1 haben, und ihre Mineralsäuresalze oder organischen, pharmazeutisch verträglichen Salze.

7. 1-[(Diarylmethoxy)alkyl] piperidine nach Anspruch 6, dadurch gekennzeichnet, daß X ein Fluoratom bedeutet, und ihre Mineralsäuresalze oder organischen, pharmazeutisch verträglichen Salze.

8. 1-[(Diarylmethoxy)alkyl] pyrrolidine gemäß Anspruch 1, dadurch gekennzeichnet, daß Alk, der Ethylenrest $-CH_2CH_2-$und n 4 ist, und ihre Mineralsäuresalze oder organischen, pharmazeutisch verträglichen Salze.

9. Die Verbindung 1-[2-(Diphenylmethoxy)ethyl]-3-(3-methoxyphenyl)pyrrolidin und ihre Mineralsäuresalze oder organischen, pharmazeutisch vertränglichen Salze.

10. Die Verbindung 1-[2-[Bis(4-fluorphenyl)methoxy]ethyl]-3-(3-methoxyphenyl)pyrrolidin und ihre Mineralsäuresalze oder organischen, pharmazeutisch verträglichen Salze.

11. Verfahren zur Herstellung der Verbindungen nach Anspruch 1, dadurch gekennzeichnet, daß man ein Verbindung der Formel

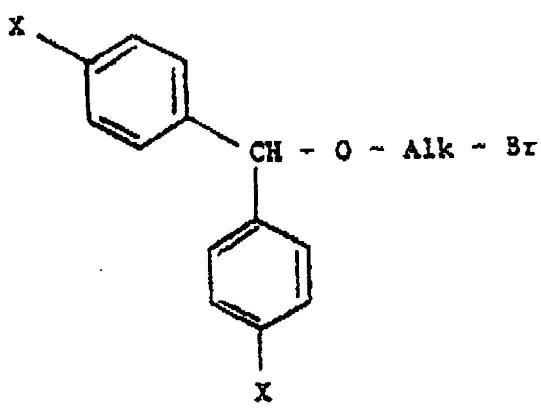

mit einem Arylpyrrolidin oder einem Arylpiperidin der Formel

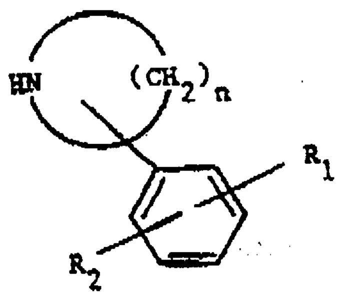

in Gegenwart eines alkalischen Mittels kondensiert, wobei X, Alk, $R_1$, $R_2$ und n die in Anspruch 1 angegebenen Bedeutungen haben.

**12.** Verfahren zur Herstellung der Verbindungen nach Anspruch 1, in denen Alk $-CH_2CH_2-$ und X = H ist, dadurch gekennzeichnet, daß man ein Säurechlorid der Formel

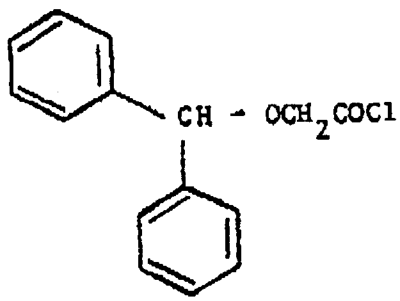

mit einem Arylpyrrolidin oder einem Arylpiperidin der Formel

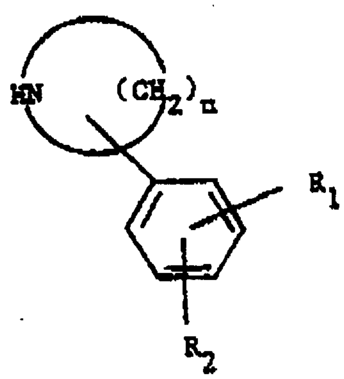

kondensiert, um ein Amid der Formel

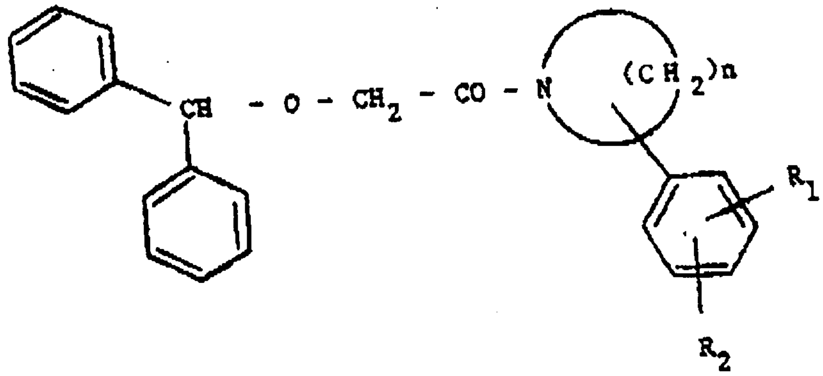

zu erhalten, das mit Lithiumhydrid und Aluminiumhydrid reduziert wird, wobei $R_1$, $R_2$ und n die in Anspruch 1 angegebenen Bedeutungen haben.

**13.** Arzneimittel, enthaltend als wirksamen Bestandteil ein 1-[(Diarylmethoxy)alkyl] pyrrolidin oder -piperidin, gekennzeichnet durch die Formel

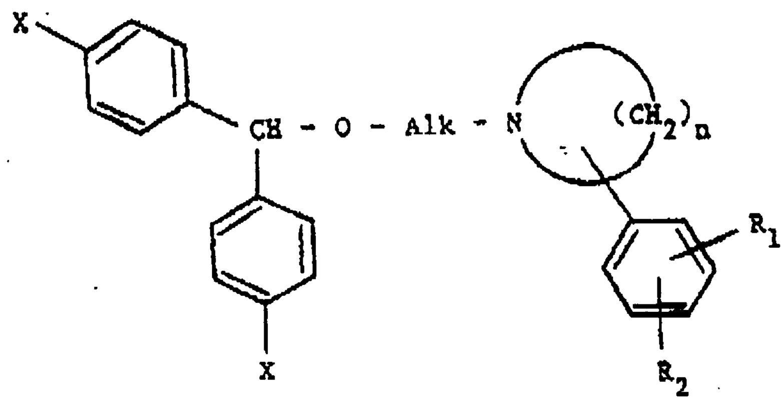

in der X Wasserstoff oder ein Fluoratom, Alk eine geradkettige oder verzweigte 2 oder 3 Kohlenstoffatome enthaltende Alkylgruppe ist; $R_1$ und $R_2$ Wasserstoff oder ein geradkettiger oder verzweigter 1 bis 4 Kohlenstoffatome enthaltender Alkoxyrest sind; n 4 oder 5 ist und seine Mineralsäuresälze oder organischen pharmazeutisch verträglichen Salze.

**Patentansprüche für folgende Vertragsstaat : ES**

**1.** Verfahren zur Herstellung von 1-[(Diarylmethoxy)alkyl] pyrrolidinen und -piperidinen, gekennzeichnet durch die Formel

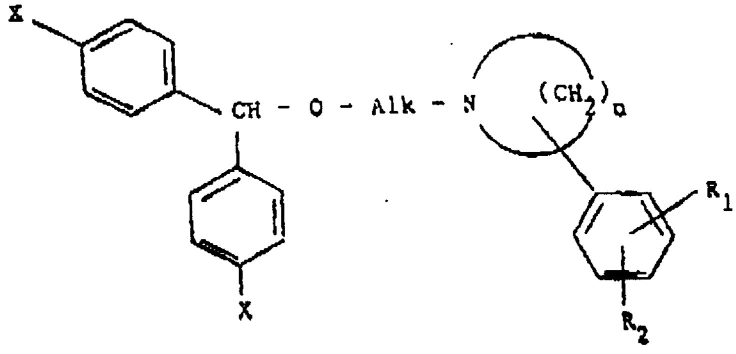

in der X Wasserstoff oder ein Fluoratom, Alk eine geradkettige oder verzweigte, 2 oder 3 Kohlenstoff-

atome enthaltende Alkylgruppe ist; $R_1$ und $R_2$ Wasserstoff oder ein geradkettiger oder verzweigter, 1 bis 4 Kohlenstoffatome enthaltender Alkoxyrest sind; n 4 oder 5 ist und ihrer Mineralsäuresalze oder organischen, pharmazeutisch verträglichen Salze, dadurch gekennzeichnet, daß man eine Verbindung der Formel

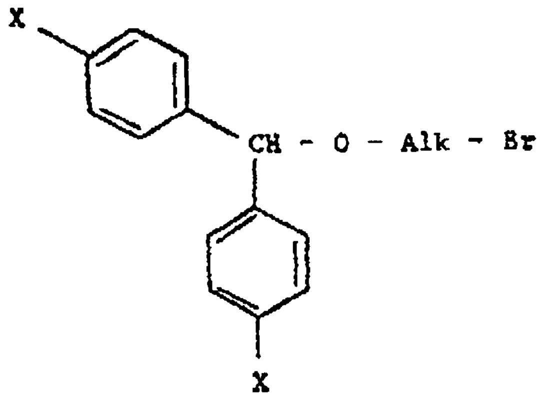

mit einem Arylpyrrolidin oder einem Arylpiperidin der Formel

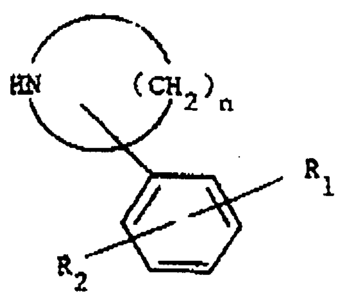

in Gegenwart eines alkalischen Mittels kondensiert, wobei X, Alk, $R_1$, $R_2$ und n die oben angegebenen Bedeutungen haben.

**2.** Verfahren zur Herstellung der Verbindungen nach Anspruch 1, in denen Alk $-CH_2CH_2-$ und X = H ist, dadurch gekennzeichnet, daß man ein Säurechlorid der Formel

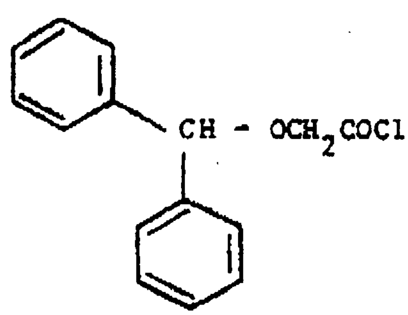

mit einem Arylpyrrolidin oder einem Arylpiperidin der Formel

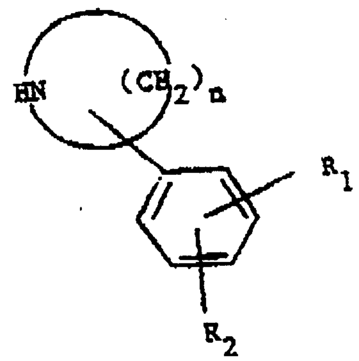

kondensiert, um ein Amid der Formel

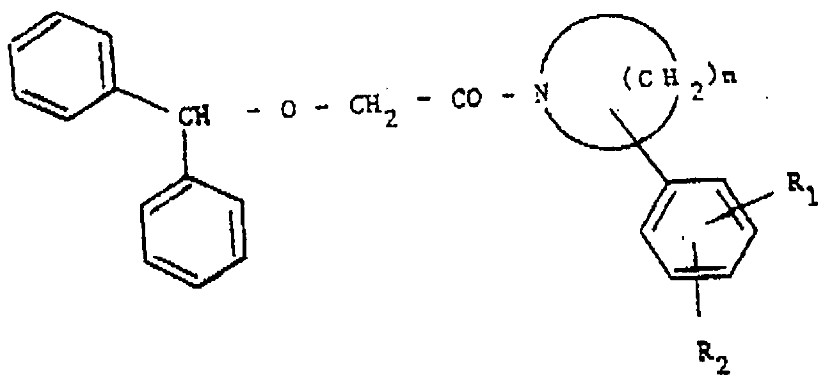

zu erhalten, des durch Lithiumhydrid und Aluminiumhydrid reduziert wird, wobei $R_1$, $R_2$ und n die in Anspruch 1 angegebene Bedeutung haben.

3. Verfahren nach Anspruch 1, dadurch gekennzeichnet, daß man 1-[(Diarylmethoxy)alkyl] pyrrolidine und -piperidine herstellt, in denen Alk ein geradkettiger, 3 oder 4 Kohlenstoffatome aufweisender Rest ist, und ihre Mineralsäuresalze oder organischen, pharmazeutisch verträglichen Salze.

4. Verfahren nach Anspruch 1, dadurch gekennzeichnet, daß man 1-[(Diarylmethoxy)alkyl] pyrrolidine und -piperidine herstellt, in denen Alk der Ethylenrest $-CH_2CH_2-$ ist, und ihre Mineralsäuresalze oder organischen, pharmazeutisch verträglichen Salze.

5. Verfahren nach Anspruch 1, dadurch gekennzeichnet, daß man 1-[(Diarylmethoxy)alkyl] pyrrolidine der allgemeinen Formel

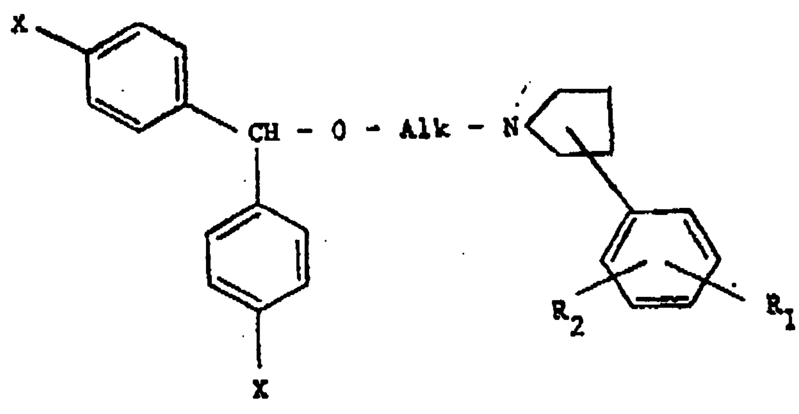

herstellt, in der X, Alk, $R_1$ und $R_2$ wie in Anspruch 1 definiert sind, und ihre Mineralsäuresalze oder

organischen, pharmazeutisch varträglichen Salze.

6. Verfahren noch Anspruch 5, dadurch gekennzeichnet, daß X ein Fluoratom bedeutet.

7. Verfahren nach Anspruch 1, dadurch gekennzeichnet, daß man 1-[(Diarylmethoxy)alkyl] piperidine der allgemeinen Formel

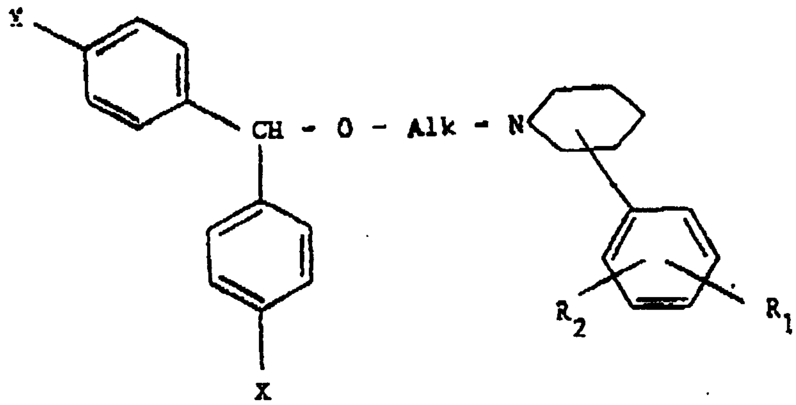

herstellt, in der X, Alk, $R_1$ und $R_2$ wie in Anspruch 1 definiert sind, und ihre Minerelsäuresalze oder organischen, pharmazeutisch verträglichen Salze.

8. Verfahren nach Anspruch 7, dadurch gekennzeichnet, daß X ein Fluoratom bedeutet.

9. Verfahren nach Anspruch 1, dadurch gekennzeichnet, daß man 1-[(Diarylmethoxy)alkyl] pyrrolidine herstellt, in denen Alk der Ethylenrest $-CH_2Ch_2-$ und n 4 ist, und ihre Mineralsäuresalze oder organischen, pharmazeutisch verträglichen Salze.

10. Verfahren nach Anspruch 1, dadurch gekennzeichnet, daß man 1-[2-(Diphenylmethoxy)ethyl]-3-(3-methoxyphenyl)pyrrolidin durch Kondensation von 1-(Diphenylmethoxy)-2-bromethan mit 3-(3-Methoxyphenyl)pyrrolidin.

11. Verfahren nach Anspruch 1, dadurch gekennzeichnet, daß man 1-[2-[Bis(4-fluorphenyl)methoxy]ethyl]-3-(3-methoxyphenyl) pyrrolidin durch Kondensation von 1-(Bis(4-fluorphenyl)methoxybromethan mit 3-(3-Methoxyphenyl)pyrrolidin in Gegenwart von Natriumhydrid.

**Patentansprüche für folgenden Vertragsstaat : GR**

1. 1-[(Diarylmethoxy)alkyl] pyrrolidine und -piperidine, gekennzeichnet durch die Formel

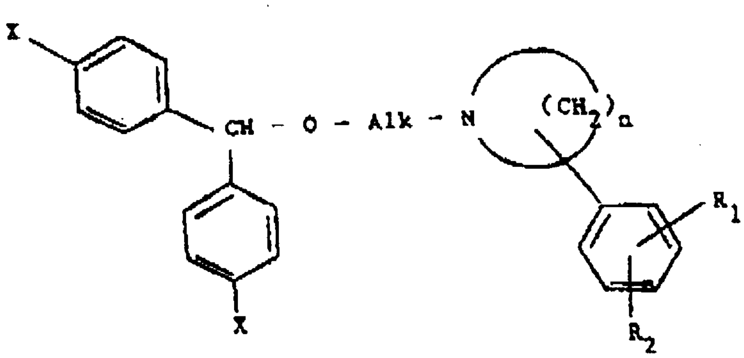

in der X Wasserstoff oder ein Fluoratom ist, Alk eine geradkettige oder verzweigte Alkylgruppe mit 2 oder 3 Kohlenstoffatomen ist; $R_1$ und $R_2$ Wasserstoff oder ein geradkettiger oder verzweigter Alkoxyrest mit 1 bis 4 Kohlenstoffatomen sind; n 4 oder 5 ist und ihre Mineralsäuresalze oder organischen, pharmazeutisch verträglichen Salze.

2. 1-[(Diarylmethoxy)alkyl] pyrrolidine und -piperidine nach Anspruch 1, dadurch gekennzeichnet, daß Alk ein geradkettiger, 2 oder 3 Kohlenstoffatome enthaltender Rest ist, und ihre Mineralsäuresalze oder organischen, pharmazeutisch verträglichen Salze.

3. 1-[(Diarylmethoxy)alkyl] pyrrolidine und -piperidine gemäß Anspruch 1, dadurch gekennzeichnet, daß Alk der Ethylenrest $-CH_2CH_2-$ ist, und ihre Mineralsäuresalze oder organischen, pharmazeutisch verträglichen Salze,

4. 1-[(Diarylmethoxy)alkyl] pyrrolidine gemäß Anspruch 1 der allgemeinen Formel

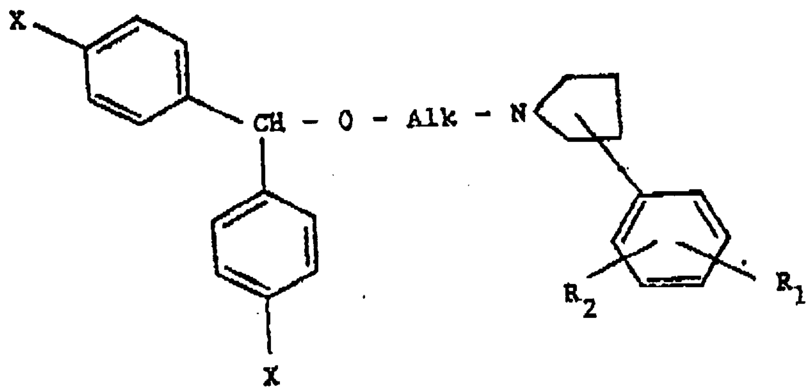

in der X, Alk, $R_1$ und $R_2$ die in Anspruch 1 angegebene Bedeutung haben, und ihre Mineralsäuresalze oder ihre organischen, pharmazeutisch verträglichen Salze.

5. 1-[(Diarylmethoxy)alkyl] pyrrolidine gemäß Anspruch 4, dadurch gekennzeichnet, daß X ein Fluoratom bedeutet, und ihre Mineralsäuresalze oder organischen, pharmazeutisch verträglichen Salze.

6. 1-[(Diarylmethoxy)alkyl] piperidine gemäß Anspruch 1 der allgemeinen Formel

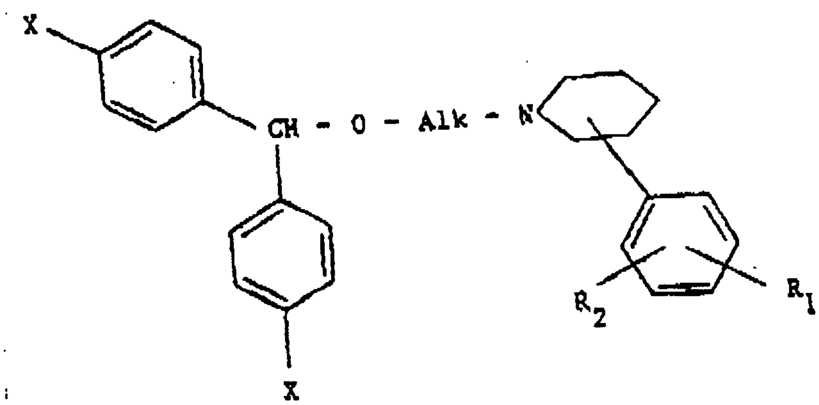

in der X, Alk, $R_1$ und $R_2$ dieselbe Bedeutung wie in Anspruch 1 haben, und ihre Mineralsäuresalze oder organischen, pharmazeutisch verträglichen Salze.

7. 1-[(Diarylmethoxy)alkyl] piperidine nach Anspruch 6, dadurch gekennzeichnet, daß X ein Fluoratom bedeutet, und ihre Mineralsäuresalze oder organischen, pharmazeutisch verträglichen Salze.

8. 1-[(Diarylmethoxy)alkyl] pyrrolidine gemäß Anspruch 1, dadurch gekennzeichnet, daß Alk, der Ethylenrest $-CH_2CH_2-$ und n 4 ist, und ihre Mineralsäuresalze oder organischen, pharmazeutisch verträglichen Salze.

9. Die Verbindung 1-[2-(Diphenylmethoxy)ethyl]-3-(3-methoxyphenyl)pyrrolidin und ihre Mineralsäuresalze oder organischen, pharmazeutisch verträglichen Salze.

10. Die Verbindung 1-[2-[Bis(4-fluorphenyl)methoxy]ethyl] -3-(3-methoxyphenyl)pyrrolidin und ihre Mineralsäuresalze oder organischen, pharmazeutisch verträglichen Salze.

11. Verfahren zur Herstellung der Verbindungen nach Anspruch 1, dadurch gekennzeichnet, daß man eine Verbindung der Formel

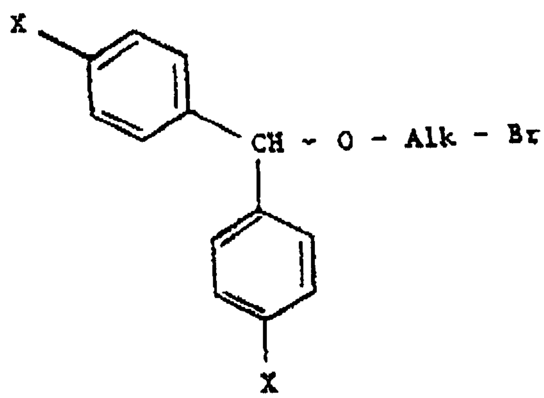

mit einem Arylpyrrolidin oder einem Arylpiperidin der Formel

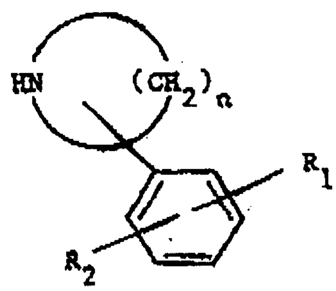

in Gegenwart eines alkalischen Mittels kondensiert, wobei X, Alk, $R_1$, $R_2$ und n die in Anspruch 1 angegebenen Bedeutungen haben.

12. Verfahren zur Herstellung der Verbindungen nach Anspruch 1, in denen Alk $-CH_2CH_2-$ und X = H ist, dadurch gekennzeichnet, daß man ein Säurechlorid der Formel

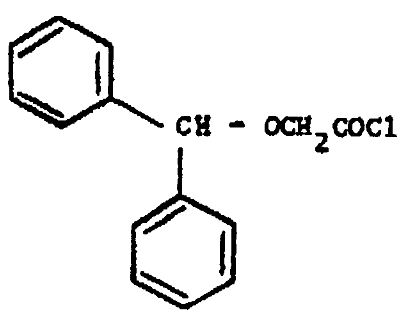

mit einem Arylpyrrolidin oder einem Arylpiperidin der Formel

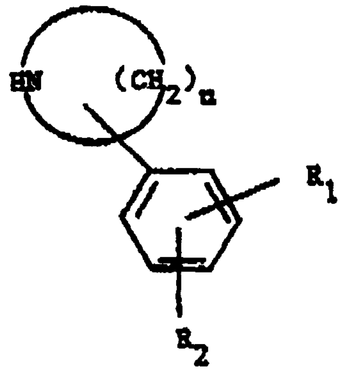

kondensiert, um ein Amid der Formel

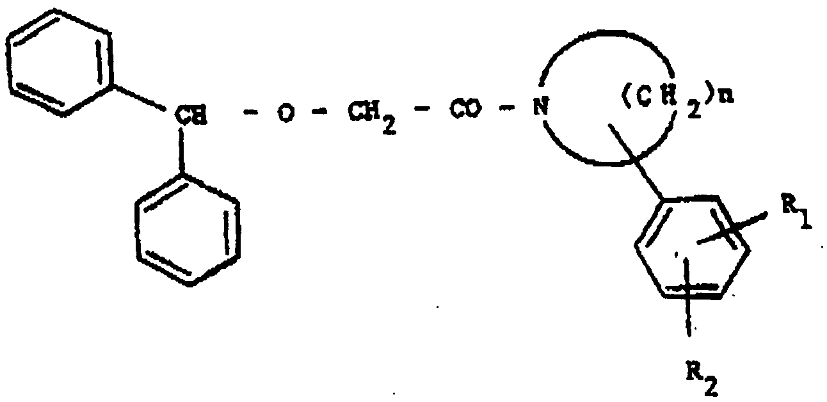

zu erhalten, das mit Lithiumhydrid und Aluminiumhydrid reduziert wird, wobei $R_1$, $R_2$ und n die in Anspruch 1 angegebenen Bedeutungen haben.